# EUROPEAN PATENT APPLICATION

(11) **EP 1 600 445 A1**
(43) Date of publication of application: **30.11.2005**
(21) Application number: 04711505.0
(22) Date of filing: 16.02.2004
(51) Int. Cl.: C07D 303/36, A61K 31/336, A61P 3/04, A61P 3/10, A61P 9/00, A61P 9/10, A61P 21/04, A61P 29/00, A61P 35/00, A61P 35/02, A61P 35/04, A61P 37/02, A61P 37/08, A61P 43/00

(54) **MEDICINAL COMPOSITION**

(30) Priority: 14.02.2003 JP 2003037167; 18.02.2003 JP 2003039098; 06.08.2003 JP 2003288281
(71) Applicant: Keio University, Tokyo 108-0073 (JP)
(72) Inventor: UMEZAWA, Kazuo, C/o Keio University, Yokohama-shi, Kanagawa, 223-8522 (JP); KATO, Kuniki C/o Keio University, Yokohama-shi, Kanagawa, 223-8522 (JP); SUZUKI, Yoshikazu, C/o Keio University, Yokohama-shi, Kanagawa, 223-8522 (JP); HORIGUCHI, Yutaka, C/o Keio University, Tokyo, 160-8582 (JP); NAKASHIMA, Jun, C/o Keio University, Tokyo, 160-8582 (JP); HATA, Hiroyuki, Kumamoto-shi, Kumamoto, 862-0912 (JP); NAMBA, Hiroyuki, C/o Nagasaki University, Nagasaki-shi, Nagasaki, 852-8523 (JP); TAKEI, Izumi, C/o Keio University, Tokyo, 160-8582 (JP); HORIE, Ryouichi, Tokyo, 157-0066 (JP)
(74) Representative: Horner, Martin Grenville
(86) International application number: PCT/JP2004/001623
(87) International publication number: WO 2004/072056

(57) **Abstract**

(-)-DHM2EQ, which is useful as an antitumor agent and an anti-inflammatory agent, can be directly obtained by optically resolving (±)-DHM2EQ using an optically active column packed with an optical resolving agent containing, for example, amylose tris(3,5-dimethylphenylcarbamate) as an active ingredient. The (-)-DHM2EQ obtained by optical resolution using the optically active column or pharmacologically acceptable salt thereof is useful as a pharmaceutical composition for improving various symptoms.

## Description

### CROSS REFERENCE TO RELATED APPLICATIONS

This application claims the benefit of priority to Japan Patent Application No. 2003-37167, filed on February 14, 2003, Japan Patent Application No. 2003-39098, filed on February 18, 2003, and Japan Patent Application No. 2003-288281, filed on August 6, 2003, all of which are incorporated herein by reference.

### TECHNICAL FIELD

The present invention relates to pharmaceutical compositions, tumor cell proliferation inhibitors, cell adhesion inhibitors, apoptosis inducers, obesity preventive and inhibitory agents, blood glucose level-lowering agents, agents for relieving inhibition of differentiation induction. This invention also relates to methods for directly separating optically active compounds, i.e., optically active substances of a 5-dehydroxymethyl derivative of epoxyquinomicin C, contained as an active ingredient in such pharmaceutical compositions and agents; and therapeutic methods using these compounds.

### BACKGROUND ART

Since there are no effective therapeutic agents for cancer, leukemia and, inflammatory diseases such as rheumatism, pancreatitis, hepatitis, and digestive system inflammation, novel chemotherapy that works by a new mechanism is expected. Novel chemotherapy that works by a new mechanism has also been awaited for immunological diseases, allergic diseases, tumor metastasis, cachexia, arteriosclerosis, neovascular diseases, etc. It has recently been revealed that NF-κB is activated in tumors or inflammation sites. The NF-κB inhibitor 5-dehydroxymethyl derivative of epoxyquinomicin C (commonly referred to as dehydroxymethylepoxyquinomicin (DHMEQ)) , recently identified, inhibits NF-κB strongly on a cellular level (A. Riga. et al., J. Biol. Chem. 277, 24625-24630, 2002), and strongly suppresses the growth of prostatic cancer (Cancer Res.2003, Jan 1; 63 (1) : 107-10) and breast cancer (a report from the Annual Meeting of the Japanese Cancer Association, 2002, p. 157) even on an organism level. DHMEQ also suppresses symptoms in a model mouse of rheumatism (N.Matsumoto et al., Bioorg. Med. Chem. Lett.10, 865-869, 2000). DHM2EQ has high specificity to these tumor cells etc and low toxicity. For this reason, DHM2EQ is expected to play a role as a novel antitumor agent and an anti-inflammatory agent.

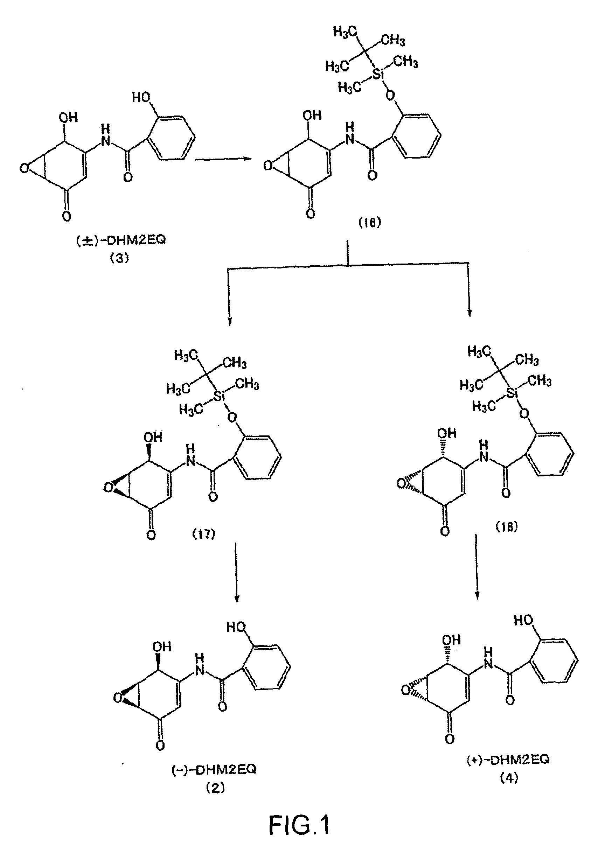

FIG. 1 shows the conventional manufacturing method of the optically active substances of DHM2EQ. First, a compound (formula (16)) in which the phenolic hydroxyl group of racemic DHM2EQ (formula (2)) has once been protected with a silyl group is resolved on a chiral column to give optically active compounds (formula (17) and formula (18)), each of which is then deprotected and intended optically active (-)-DHM2EQ (formula (2)) and (+)-DHM2EQ (formula (4)) are obtained. However, this method causes a problem of a low yield due to the long process (N. Matsumoto et al., Bioorg.Med.Chem.Lett.10, 865-869, 2000) .

Thus, a major object of the present invention is to provide methods that enable high-speed, high-yield production of compounds to be contained as effective ingredients in pharmaceutical compositions, which have a superior pharmacological effect and high specificity. Another object of the present invention is to provide pharmaceutical compositions that contain the aforementioned compound as active ingredients.

### DISCLOSURE OF THE INVENTION

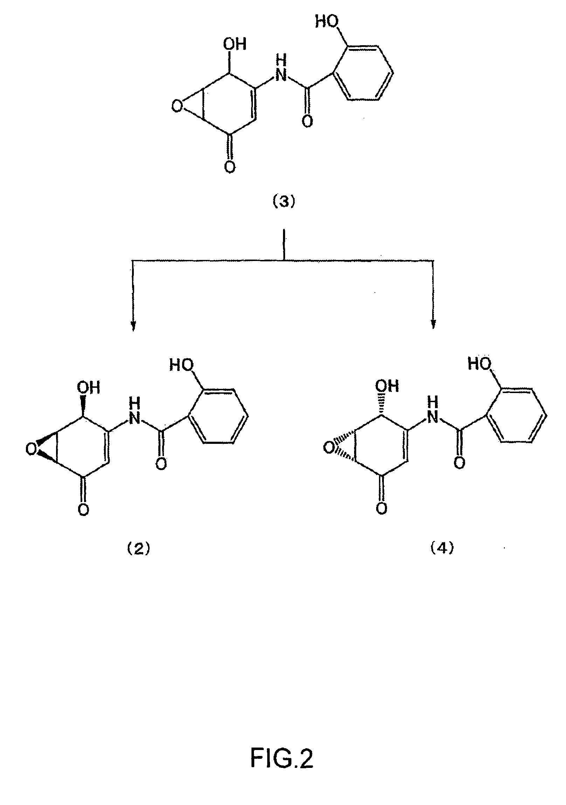

The present inventors have intensively studied to solve the above-mentioned problems and have found that a racemate of a compound represented by formula (3) can be directly resolved into optically active compounds (formulae (2) and (4)) by high performance liquid chromatography (HPLC) using an optically active column packed with a resolving agent containing as an active ingredient a polysaccharide aromatic carbamate derivative substituted with a group represented by formula (5) (FIG.2) .

Further, to examine whether the optically active compounds (formula (2) and formula (4)) have a more excellent effect than the inhibitory effect on adhesion of vascular endothelial cells exerted by a racemate of the compound represented by formula (3), the inventors investigated adhesion of human umbilical vein endothelial cells (HUVEC) to human acute myelogenous leukemia HL-60 cells using each of the compounds represented by formulae (2), (3), and (4). As a result, the inventors found that a compound represented by formula (2) exhibits a stronger inhibitory effect on adhesion of vascular endothelial cells than compounds represented by formula (3) and formula (4).

The inventors further found that a compound represented by formula (2) suppresses proliferation of tumor cells in multiple myeloma, thyroid cancer, etc.

The inventors also found that by treating hypertrophic fat cells with a compound represented with formula (2) apoptosis of the hypertrophic fat cells are induced.

The inventors also found that a compound represented by formula (2) suppressed an increase in body weight resulting from high-fat diet load in a model animal of obesity and type 2 diabetes.

In addition, the inventors found that a compound represented by formula (2) relieves inhibition of differentiation induction from myoblasts to muscle cells in cultured cells. The inventors have thus accomplished the present invention.

Thus, the pharmaceutical composition according to the present invention for improving a symptom accompanied by activation of NF-κB contains an optically active compound represented by the following general formula (1) or a pharmacologically acceptable salt thereof as an active ingredient. 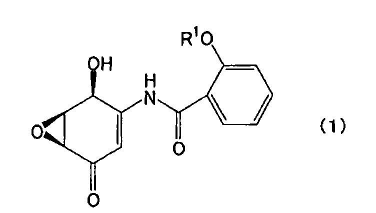 wherein R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group.

The aforementioned compound is preferably the following formula (2).

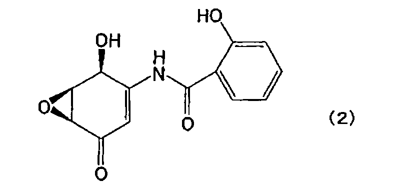

The pharmaceutical composition according to the present invention improves at least one symptom by causing apoptosis of a tumor cell and improves at least one symptom resulting from a tumor cell without the contribution of apoptosis of the tumor cell.

The aforementioned symptom accompanied by activation of NF-κB may result from, for example, a tumor cells. The aforementioned symptom is improved by inhibiting proliferation of the aforementioned tumor cell.

The aforementioned symptom is also improved by inhibiting adhesion of a tumor cell to a vascular endothelial cell.

The aforementioned symptom is one selected from the group consisting of an immunological disease, an allergic disease, an inflammatory disease, tumor metastasis, cachexia, arteriosclerosis, a nonvascular diseases (intratumoral nonvascular disease), and leukemia.

The aforementioned tumor is illustratively myeloma, thyroid cancer, breast cancer, pancreatic cancer, a malignant tumor, prostatic cancer, etc.

The pharmaceutical composition according to the present invention contains as an active ingredient an optically active compound represented by the following general formula (1), which is capable of enhancing the effect of a therapy by inhibiting activation of NF-κB caused by the therapy that causes the activation of NF-κB, or a pharmacologically acceptable salt thereof. 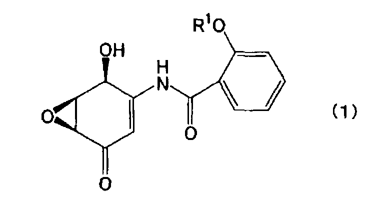 wherein R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group.

The aforementioned compound is preferably the following formula (2).

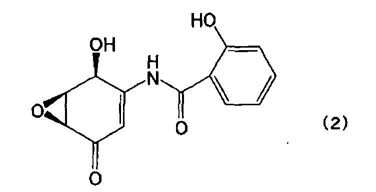

The therapy that activates NF-κB may be a therapy using an antitumor agent or radiotherapy for a tumor cell. It should be noted that the pharmaceutical composition may contain the antitumor agent as an active ingredient. The antitumor agent is illustratively camptothecin or daunorubicin.

Further, the pharmaceutical composition according to the present invention for improving a disease caused by TNF-α contains an optically active compound represented by the following general formula (1) or a pharmacologically acceptable salt thereof as an active ingredient. 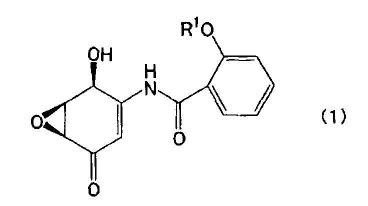 wherein R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group. The aforementioned compound is preferably the following formula (2).

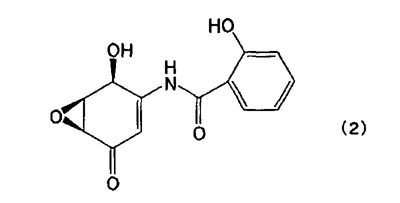

The aforementioned disease caused by TNF-α may be a disease involving insulin resistance, a disease resulting from diabetes, a muscular dystrophy, etc.

It should be noted that the above-mentioned pharmaceutical composition contains a (-)-compound with a superior pharmacological effect as an active ingredient and that a composition that substantially does not contain a (+)-compound is particularly preferred.

The tumor cell proliferation inhibitor for inhibiting proliferation of a tumor cell according to the present invention contains an optically active compound represented by the following general formula (1) or a pharmacologically acceptable salt thereof as an active ingredient. 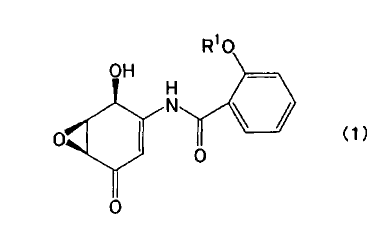 wherein R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group.

It should be noted that the tumor cell proliferation inhibitor for inhibiting proliferation of a tumor cell according to the present invention contains a (-) -compound with a superior pharmacological effect as an active ingredient and that an inhibitor that substantially does not contain a (+)-compound is particularly preferred.

The aforementioned compound may be following formula (2) .

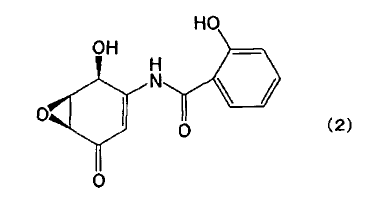

The adhesion molecule expression suppressor for suppressing expression of an adhesion molecule in a vascular endothelial cell according to the present invention contains an optically active compound represented by the following general formula (1) or a pharmacologically acceptable salt thereof as an active ingredient. 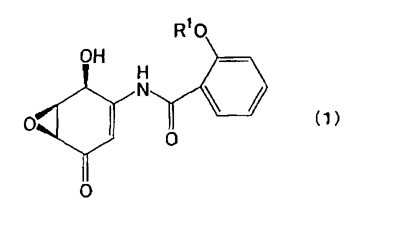 wherein R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group.

It should be noted that the adhesion molecule expression suppressor for suppressing expression of an adhesion molecule in a vascular endothelial cell according to the present invention contains a (-)-compound with a superior pharmacological effect as an active ingredient and that a suppressor that substantially does not contain a (+)-compound is particularly preferred.

The aforementioned compound may be following formula (2) .

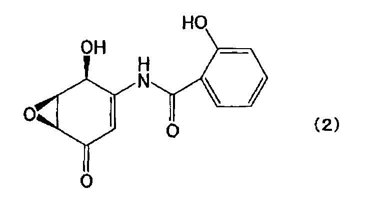

The apoptosis inducer for inducing apoptosis of a tumor cell according to the present invention contains an optically active compound represented by the following general formula (1) or a pharmacologically acceptable salt thereof as an active ingredient. 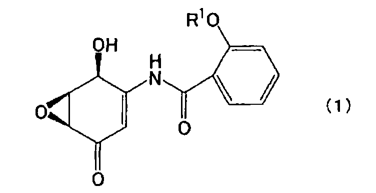 wherein R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group.

It should be noted that the apoptosis inducer for inducing apoptosis of a tumor cell according to the present invention contains a (-)-compound with a superior pharmacological effect as an active ingredient and that an inducer that substantially does not contain a (+)-compound is particularly preferred.

The aforementioned compound may be following formula (2) .

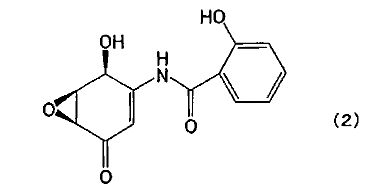

The apoptosis inducer for inducing apoptosis of a hypertrophic fat cell according to the present invention contains an optically active compound represented by the following general formula (1) or a pharmacologically acceptable salt thereof as an active ingredient. 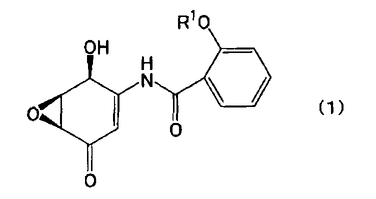 wherein R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group. The aforementioned compound may be following formula (2) .

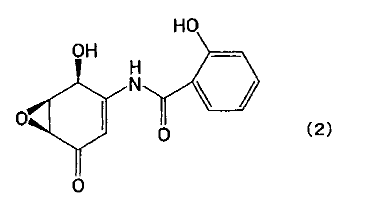

It should be noted that the apoptosis inducer for inducing apoptosis of a hypertrophic fat cell according to the present invention contains a (-)-compound with a superior pharmacological effect as an active ingredient and that an inducer that substantially does not contain a (+) -compound is particularly preferred. In addition, the apoptosis inducer for inducing apoptosis of a hypertrophic fat cell according to the present invention may further contain TNF-α as an active ingredient.

The obesity preventive and inhibitory agent according to the present invention contains an optically active compound represented by the following general formula (1) or a pharmacologically acceptable salt thereof as an active ingredient. 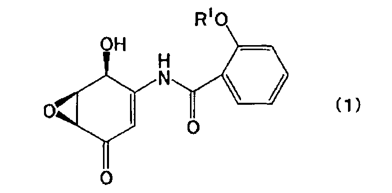 wherein R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group. The aforementioned compound may be following formula (2).

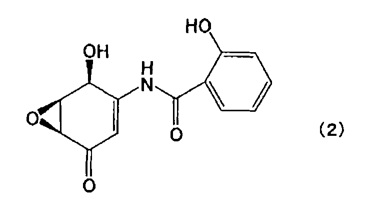

It should be noted that the obesity prevention and inhibition agent according to the present invention contains a (-)-compound with a superior pharmacological effect as an active ingredient and that an agent that substantially does not contain a (+)-compound is particularly preferred.

The blood glucose level-lowering agent according to the present invention contains an optically active compound represented by the following general formula (1) or a pharmacologically acceptable salt thereof as an active ingredient. 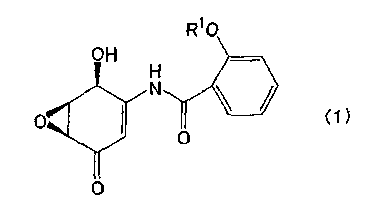 wherein R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group. The aforementioned compound may be following formula (2).

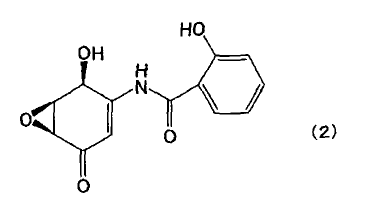

It should be noted that the blood glucose level-lowering agent according to the present invention contains a (-)-compound with a superior pharmacological effect as an active ingredient and that an agent that substantially does not contain a (+)-compound is particularly preferred.

The agent for relieving inhibition of induction of cell differentiation according to the present invention contains an optically active compound represented by the following general formula (1) or a pharmacologically acceptable salt thereof as an active ingredient. 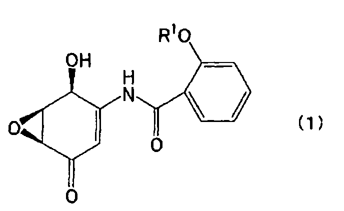 wherein R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group. The aforementioned compound is preferably the following formula (2).

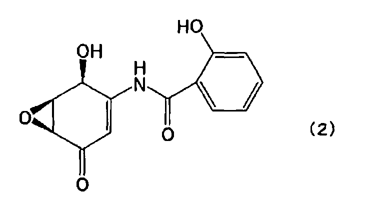

It should be noted that the inhibition of induction of cell differentiation may be suppression of muscle cell differentiation by TNF-α. The agent for relieving inhibition of induction of cell differentiation according to the present invention contains a (-)-compound with a superior pharmacological effect as an active ingredient and that an agent that substantially does not contain a (+)-compound is particularly preferred.

The present invention can also provide a method for producing an optically active compound represented by the following formula (2) or (4), by directly optically resolving a racemate of a compound represented by formula (3). 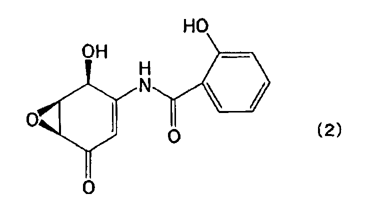 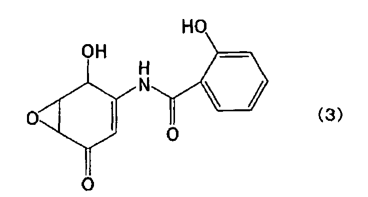 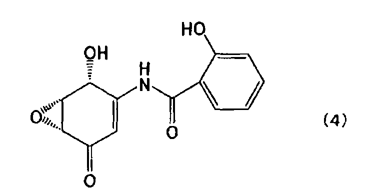 The aforementioned optical resolution may be performed using an optically active column.

The aforementioned optically active column may be packed with a resolving agent containing as an active ingredient a polysaccharide aromatic carbamate derivative substituted with a group represented by the following formula (5). 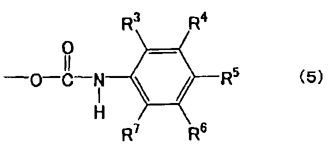 wherein R³ to R⁷ may each independently represent a hydrogen atom, an alkyl group having 1 to 8 carbon atoms, an alkoxy group having 1 to 8 carbon atoms, an aromatic group having 6 to 14 carbon atoms, or a halogen atom, etc.

The aforementioned polysaccharide aromatic carbamate derivative may be amylose tris (3,5-dimethylphenylcarbamate) or cellulose tris (3,5-dimethylphenylcarbamate). A DAICEL CHIRALPAK AD column (Daicel Chemical Industries, Ltd.) packed with which a resolving agent containing amylose tris (3, 5-dimethylphenylcarbamate) as an active ingredient is particularly preferred.

The method according to the present invention enables high-efficiency mass production of optically active DHMEQ.

The method according to the present invention makes it possible to produce optically active DHMEQ represented by formula (2) or (4) . Of these two optically active substances, the compound represented by formula (2) is more biologically active (pharmacologically active) than a compound represented by formula (4), as shown in the Examples described later. The direct resolution method according to the present invention may be performed by directly optically resolving a racemate of a compound represented by formula (3) using a resolving agent containing as an active ingredient a polysaccharide aromatic carbamate derivative substituted with a group represented by the following formula (5). 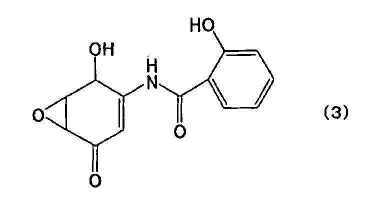 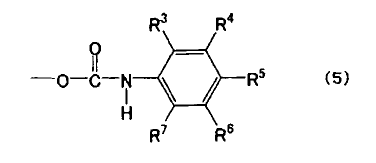 wherein R³ to R⁷ may each independently represent a hydrogen atom, an alkyl group having 1 to 8 carbon atoms, an alkoxy group having 1 to 8 carbon atoms, an aromatic group having 6 to 14 carbon atoms, or a halogen atom, etc.

The aforementioned polysaccharide aromatic carbamate derivative may be amylose tris (3, 5-dimethylphenylcarbamate) or cellulose tris (3, 5-dimethylphenylcarbamate).

The therapeutic method according to the present invention uses an optically active compound represented by the following general formula (1) or a pharmacologically acceptable salt thereof for improving a disease accompanied by activation of NF-κB. 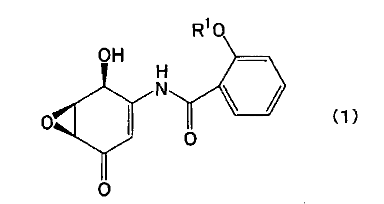 wherein R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group.

The aforementioned compound is preferably the following formula (2).

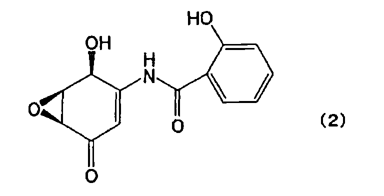

The therapeutic method according to the present invention may improve at least one symptom by causing apoptosis of a tumor cell and improve at least one symptom resulting from a tumor cell without the contribution of apoptosis of the tumor cells.

The aforementioned symptom accompanied by activation of NF-κB may result, for example, from a tumor cell.

The aforementioned symptom may be improved by inhibiting proliferation of the aforementioned tumor cell or by inhibiting adhesion of a tumor cell to a vascular endothelial cell

The aforementioned symptom is one selected from the group consisting of an immunological disease, an allergic disease, an inflammatory disease, tumor metastasis, cachexia, arteriosclerosis, a nonvascular diseases, (intratumoral nonvascular disease), and leukemia.

The aforementioned tumor is illustratively myeloma, thyroid cancer, breast cancer, pancreatic cancer, malignant tumors, prostatic cancer, etc.

The therapeutic method according to the present invention uses a pharmaceutical composition containing as an active ingredient an optically active compound represented by the following general formula (1) or a pharmacologically acceptable salt thereof, which is capable of enhancing the effect of a therapy by inhibiting activation of NF-κB caused by the therapy that causes the activation of NF-κB, 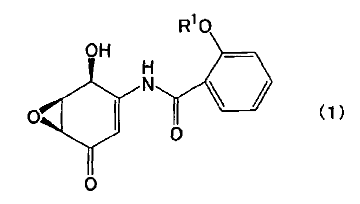 wherein R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group.

The aforementioned compound is preferably the following formula (2).

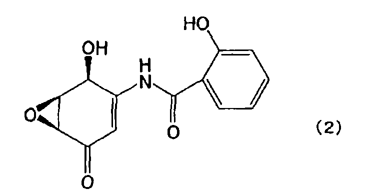

The aforementioned therapy that activates NF-κB is a therapy using an antitumor agent or radiotherapy for a tumor cell. It should be noted that the pharmaceutical composition may contain the antitumor agent as an active ingredient. The antitumor agent is illustratively camptothecin or daunorubicin.

The therapeutic method according to the present invention uses an optically active compound represented by the following general formula (1) or a pharmacologically acceptable salt thereof for improving a disease caused by TNF-α. 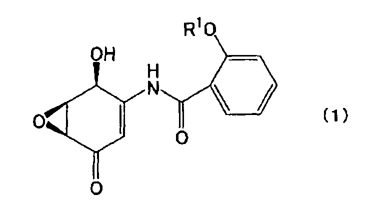 wherein R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group. The aforementioned compound is preferably the following formula (2) .

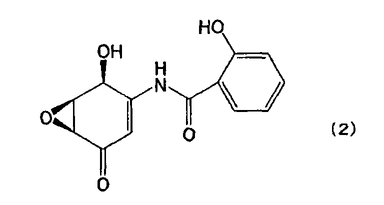

The aforementioned disease caused by TNF-α is a disease involving insulin resistance, a disease resulting from diabetes, a muscular dystrophy, etc.

It should be noted that the above-mentioned therapeutic methods according to the present invention use a (-)-compound with a superior pharmacological effect as an active ingredient and that a method that substantially does not use a (+) -compound is particularly preferred.

### BRIEF DESCRIPTION OF THE DRAWINGS

FIG. 1 shows the reaction scheme of the conventional method for producing optically active substances of DHM2EQ.
FIG. 2 shows the reaction scheme of the method for producing optically active DHM2EQ (formula (2) and formula (4)) according to the present invention.
FIG. 3 shows HPLC profiles of (-) -DHM2EQ and (+)-DHM2EQ.
FIG. 4 shows the suppressive effect of (-) -DHM2EQ exerted on adhesion of HUVECs to HL-60 cells.
FIG. 5 shows the antitumor effect of (-)-DHM2EQ exerted on myeloma model mice.
FIG. 6 shows the antitumor effect of (-)-DHM2EQ exerted on thyroid cancer model mice.
FIG. 7 shows a result of analysis of the apoptosis-inducing effect of (-)-DHM2EQ on hypertrophic fat cells.
FIG. 8 shows the suppressive effect of (-)-DHM2EQ exerted on weight gain in obesity-associated diabetes model mice.
FIG. 9 shows the effect in which (-)-DHM2EQ relieves inhibition of differentiation induction from myoblasts to muscle cells.

### BEST MODE FOR CARRYING OUT THE INVENTION

The Examples according to the present invention are hereinafter described in detail. Unless otherwise explained, methods described in standard sets of protocols such as J. Sambrook and E. F. Fritsch & T. Maniatis (Ed.), "Molecular Cloning, a Laboratory Manual (3rd edition), Cold Spring HarborPress and Cold Spring Harbor, New York (2001) ; and F. M. Ausubel, R. Brent, R. E. Kingston, D. D. Moore, J. G. Seidman, J. A. Smith, and K. Struhl (Ed.), "Current Protocols in Molecular Biology," John Wiley & Sons Ltd., or their modified and/or changed methods are used. When using commercial reagent kits and measuring apparatus, unless otherwise explained, their attached protocols are used.

The objective, characteristics, and advantages of the present invention as well as the idea thereof will be apparent to those skilled in the art from the descriptions given herein. It is to be understood that the embodiments and specific examples of the invention described hereinbelow are to be taken as preferred examples of the present invention. These descriptions are for illustrative and explanatory purposes only and are not intended to limit the invention to these embodiments or examples. It is further apparent to those skilled in the art that various changes and modifications may be made based on the descriptions given herein within the intent and scope of the present invention disclosed herein.

NF-κB is a pivotal transcription factor involved in host defense responses. It is known that many genes induced by NF-κ B are deeply involved in immune responses or inflammatory reactions of, besides immunoglobulins, cytokines (IL (interleukin)-1, IL-2, IL-6, IL-8, TNF(tumor necrosis factor)-α, etc.), cell adhesion factor (E-selectin, ICAM-(intercellular adhesion molecule) 1, VCAM(vascular cell adhesion molecule)-1, etc.),nitric oxide (NO) synthase, Fas ligand, etc (Cell, 807, 13-20, 1996). It is considered that constitutive activation of NF-κB that has been reported for bladder cancer, breast cancer, melanoma, etc. is involved in various aspects of tumorigenesis including suppression of apoptosis, promotion of cell proliferation, suppression of cell differentiation, etc., thus promoting tumorigenesis (J. Clin. Invest. 107, 241-246, 2001) . Meanwhile, as it has been reported that gene transfer of IκB, an NF-κB activation inhibitory protein, specifically induces cell death, compounds having the inhibitory effect on NF-κB activation are expected to serve as the drugs or drugs with anti-inflammatory activity and antitumor activity.

The previously known compounds that have an inhibitory effect on NF-κB activation include salicylamide derivatives (WO01/12588 A1), panepoxydone (Biochem. Biophys. Res. Commun. 226, 214-221, 1996), cycloepoxydon (J. Antibiot. 51, 455-463, 1998), SN-50 (J. Biol. Chem. 270, 14255-14258, 1995), etc.

The above-mentioned salicylamide derivatives were newly designed based on the structure of nontoxic antibiotic epoxyquinomicin. The inventors has recently clarified that a racemate of this compound has an anti-inflammatory effect, an immunosuppressive effect, antitumor activity, an anti-arteriosclerosis effect, a suppressive effect on tumor metastasis, an anti-cachexia effect, an antiallergic effect, an inhibitory effect on angiogenesis, etc.

A racemate is a mixture of two enantiomers ((+)- and (-)-) , which generally have almost identical physical properties such as the boiling point and the melting point. However, a (+)-compound and a (-)-compound can have different effects typically in vivo. For example, like "the tragedy of thalidomide," it is known that one enantiomer has hypnotic and sedative effects whereas the other enantiomer has a teratogenic effect (the effect of inducing malformations in the fetus) . In drugs, agricultural chemicals, etc., it is also known that the use of an agent containing one enantiomer from a racemate as an active ingredient produces more beneficial effect with fewer side effects. Therefore, in drugs etc., preparation of an optically pure compound has been an extremely important problem to be solved.

The inventors have already attempted to resolve a racemate of the aforementioned salicylamide derivatives into a (+)-compound and a (-)-compound (Bioorg. Med. Chem. Lett.10, 865-869, 2000). However, the method for resolving the aforementioned compound required preparation of a compound in which the phenolic hydroxyl group of a racemate had once been protected with a silyl group. Thus, the inventors investigated whether a racemate of the aforementioned compound can be directly resolved into a (-)- compound and a (+)- compound using an optically active column packed with a resolving agent containing various derivatives as active ingredients. As a result, they found that a racemate of the aforementioned compound can be directly resolved into optically active compounds using a resolving agent containing as an active ingredient a polysaccharide aromatic carbamate derivative substituted with a group represented by formula (6).

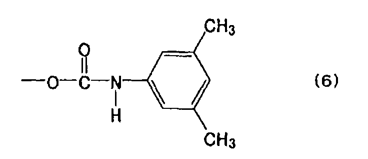

It should be noted that a substituent sometimes changes the form of a molecule or physical and chemical properties possessed by a substituent itself and electronic influences of the substituent on the n electron system of the aromatic ring are complicatedly intermingled. For this reason, considering the relationship between substitution and significant changes in the properties of separation or adsorption, the substituents (-R³, -R⁴, -R⁵, -R⁶, and -R⁷) represented by the following general formula (5) may be each independently a hydrogen atom, an alkyl group having 1 to 8 carbon atoms, an alkoxy group having 1 to 8 carbon atoms, an aromatic group having 6 to 14 carbon atoms, or a halogen atom, etc.

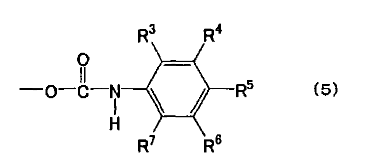

The polysaccharide used as the starting material of a polysaccharide aromatic carbamate derivative may be any one of a synthetic polysaccharide, a natural polysaccharide, a modified natural polysaccharide, preferably an optically active polysaccharide, more preferably amylose, cellulose, etc., which provide a high-purity polysaccharide. Other examples of polysaccharides from which high purity polysaccharide are readily available, and which may be used as starting materials include β-1,4-chitosan, chitin, β-1, 4-mannan, β-1,4-xylan, inulin, curdlan, etc. In the Examples herein, amylose tris (3, 5-dimethylphenylcarbamate) are used as a polysaccharide aromatic carbamate derivative in performing the direct resolution according to the present invention, but cellulose tris (3, 5-dimethylphenylcarbamate) may be used.

In the foregoing, explanation has been made of polysaccharide carbamate derivatives to be contained as an active ingredient in the resolving agent, but other polysaccharide derivatives, such as ester derivatives, ether derivatives, carbamate derivatives other than an aromatic carbamate may be used.

As a carrier that supports these resolving agents, either an organic carrier or an inorganic carrier is generally used, of which an inorganic carrier is more preferred. Examples of such an inorganic carrier include silica gel, alumina, magnesia, titanium oxide, glass, silicate, kaoline, etc. The particle size of the carrier varies with the size of the column to be used, but generally ranges from 0.5 µm to 10 mm, preferably from 1 µm to 300 µm, and more preferably from 5 µm to 20 µm. In addition, the carrier preferably has a porous property and the average pore size of a porous carrier is preferably 10 Å to 100 µm, more preferably 50 Å to 50000 Å. As the method of having the aforementioned polysaccharide aromatic carbamate derivative carried on a carrier, general methods such as one for, e.g., dissolving the polysaccharide aromatic carbamate derivative in a solvent to form a dope solution, which is slowly dripped onto the carrier while being stirred, so that the carrier is uniformly coated with the dope.

By applying the above-mentioned direct resolution method, it is also possible to produce optically active compounds (formula (2) and formula (4)) in a simple and convenient manner from a racemate of a compound represented by formula (3) . The methods for producing the compounds according to the present invention are described in detail hereinbelow.

### === Method for Producing Compounds Represented by Formulae (2) and (4) ===

The compounds represented by the general formula (1) can be produced according to the synthetic process by Wipf et al. (Synthesis, No. 12, p. 1549-1561, 1995).

One example of the processes for producing compounds represented by the general formula (12) will be illustrated hereinbelow, based on the following reaction schemes.

In the following reaction scheme, R¹ represents a hydrogen atom or a C2-4 alkanoyl group. Examples of the alkanoyl group include, an acetyl group, a propionyl group, and a butanoyl group, together with isomer groups thereof, and particularly preferred among these is an acetyl group. R² represents C1-4 alkyl group. Examples of the alkyl group include, a methyl group, an ethyl group, a propyl group, a butyl group, together with isomer groups thereof, and preferred among these are a methyl group and an ethyl group, particularly a methyl group. X represents a halogen atom. Examples of the halogen atom include fluorine, chlorine, bromine and iodine atoms, and preferred among these are chlorine and bromine atoms, in particular a chlorine atom.

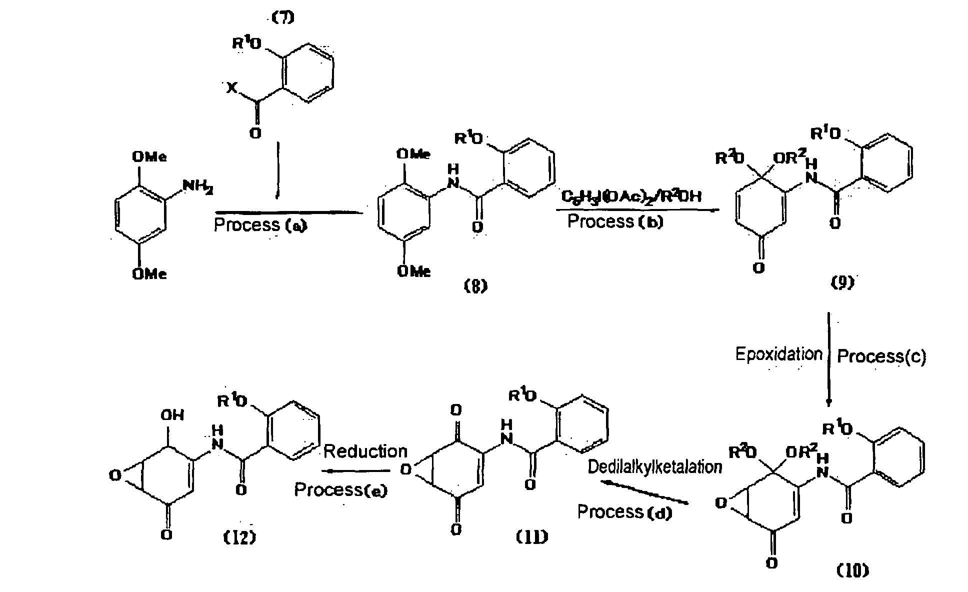

### process a: Preparation of N-(2-alkanoylbenzoyl)-2,5-dimethoxyaniline

2, 5-Dimethoxyaniline is dissolved in a solvent (pyridine, etc.), and ethyl acetate solution of O-alkanoylsalicyloyl halide of formula (7) is added thereto at -78°C to 50°C, preferably under ice cooling, and the mixture is reacted while stirring. After stopping the reaction by addition of water, ethyl acetate is added to the reaction mixture, which then is sequentially washed with hydrochloric acid, water, a sodium hydrogencarbonate solution and water. After drying, the organic layer is concentrated under reduced pressure and dried under vacuum to obtain an N-(2-alkanoylbenzoyl)-2,5-dimethoxyaniline compound represented by formula (2). The compound can be used in the next process without purification.

### Process b: Preparation of 3-(O-alkanoylsalicyloyl) amino-4,4-dialkoxy-2,5-cyclohexadienone

The compound of formula (8) obtained as described above is dissolved in a solvent such as methanol, diacetoxyiodobenzene is added thereto at -20°C to 50°C, preferably under ice cooling and the mixture is reacted at room temperature while stirring. After concentration under reduced pressure, ethyl acetate is added and the reaction mixture is washed with sodium hydrogencarbonate solution and saline. Then, the solvent is concentrated under reduced pressure and the obtained residue is purified by column chromatography to obtain 3-(O-alkanoylsalicyloyl) amino-4,4-dialkoxy-2,5-cyclohexadienone.

### Process c: Preparation of 5,6-epxoy-4,4-dialkoxy-3-salicyloylamino-2-cyclohexenone compound

3-(O-Alkanoylsalicyloyl)amino-4,4-dialkoxy-2,5-cycloh exadienone represented by formula (9) is dissolved in a solvent (tetrahydrofuran, methanol, etc.), hydrogen peroxide water and sodium hydroxide are added thereto at -20°C to 50°C, preferably under ice cooling, and the mixture is reacted while stirring. Ethyl acetate is added to the reaction mixture, which is sequentially washed with hydrochloric solution, aqueous sodium thiosulfate solution, and saline. After drying in the air, the reaction mixture is dried under vacuum. In order to remove the residual starting compound, the residue is dissolved in acetone; p-toluenesulfonic acid is added thereto and stirred at room temperature to decompose the starting compound. Ethyl acetate is added to the residue obtained by distilling off methanol under reduced pressure, and the solution is washed with water. The residue obtained by drying the ethyl acetate layer is purified by column chromatography to obtain 5,6-epxoy-4,4-dialkoxy-3-salicyloylamino-2-cyclohexenone compound represented by formula (10) .

### Process d: Preparation of 5,6-epoxy-2-salicyloylamino-2cyclohexen-1,4dione

5,6-Epxoy-4,4-dialkoxy-3-salicyloylamino-2-cyclohexen one compound represented by formula (10) is dissolved in methylene chloride, an inorganic acid or organic acid (trifluoroboron diethyl ether complex, etc.) is added under ice cooling, and the mixture is reacted while stirring. A solvent (ethyl acetate, etc.) is added to the reaction mixture, which is washed with water. After concentrating the ethyl acetate layer, the obtained residue is washed with methanol to obtain 5,6-epoxy-2-salicyloylamino-2-cyclohexen-1,4-dione represented by formula (11).

### Process e: Preparation of 5,6-epoxy-4-hydroxy-3-salicyloylamino-2-cyclohexenone

5,6-Epoxy-2-salicyloyalamino-2-cyclohexen-1,4-dione represented by formula (5) is suspended in a solvent (methanol, ethanol, THF, etc.) and a reducing agent (sodium borohydride, etc.) is added thereto at -78°C to 50° C, preferably under ice cooling. A solvent (ethyl acetate, methylene chloride, etc.) is added to the reaction mixture, which is sequentially washed with hydrochloric acid and water. After drying, the solvent layer is concentrated under reduced pressure, suspended, stirred and washed with methanol to obtain 5,6-epoxy-4-hydroxy-3-salicyloylamino-2-cyclohexenone (DHM2EQ) represented by formula (12).

It should be noted that by using O-acetylsalicyloyl chloride as O-alkanoylsalicyloyl halide of formula (7) as well as using methanol as a solvent for dissolving a compound of formula (8) a compound represented by formula (3) can be produced. ((±)-DHM2EQ; it should be noted that "DHM2EQ" is in some cases referred to as "DHMEQ.")

Optical resolution of a compound of formula (3) is performed by high performance liquid chromatography (HPLC) using an optically active column, whereby compounds of formula (2) and formula (4) can be obtained. As an optically active column, a resolving agent containing as an active ingredient a polysaccharide aromatic carbamate derivative substituted with a group represented with the general formula (5), for example, a DAICEL CHIRALPAK AD column (10 mm i.d. x 250 mm) can be used. When a DAICEL CHIRALPAK AD column is used, compounds of formulae (2) and (4) may be isolated by using 0.1-0.9 v/v% acetic acid in methanol as a mobile phase (e.g., at a flow rate of 0.5-2.5 ml/min), preparing a methanol solution (e.g., at a concentration of 5-20 mg/ml) of a compound represented by formula (3), and injecting the solution in 5-20 portions (50-200 µl) .

By measuring optical rotations of compounds of formulae (2) and (4) with a JASCO DIP-360 polarimeter, their optical purities can be determined. Since the optical rotation of a compound represented by formula (2) is levorotatory, (-)- is added to the name of the compound. Since the optical rotation of a compound represented by formula (4) is dextrorotatory, (+)- is added to the name of the compound.

### === About the pharmacological effects of a compound represented by formula (2)===

(-)-DHM2EQ can improve (prevent and suppress progression of) symptoms accompanied by activation of NF-κB, specifically, such as symptoms resulting from tumor cells (e.g., breast cancer, pancreatic cancer, malignant tumors of the lymphoid system, prostatic cancer, thyroid cancer, myeloma, etc.); symptoms of immunological disease, allergic disease, inflammatory disease, tumor metastasis, cachexia, arteriosclerosis, neovascular disease (intratumoral neovascular disease in particular), leukemia (adult T-cell leukemia in particular), etc; disease with insulin resistance; disease derived from diabetes; and muscular dystrophy, with a more beneficial effect than that of (±)-DHM2EQ.

Therefore, when used for tumor cell proliferation inhibitors, adhesion molecule expression inhibitors, apoptosis inducers, obesity preventive and inhibitory agents, blood glucose level-lowering agents, agents for relieving inhibition of differentiation induction, etc., (-)-DHM2EQ is more useful than (±)-DHM2EQ.

To improve symptoms resulting from tumor cells, apoptosis of tumor cells may or may not make a contribution. Examples of the case in which apoptosis does not make a contribution include, but are not limited to, inhibition of tumor metastasis by inhibition of cell adhesion, tumor growth inhibition not involving apoptosis, improvement of cancer cachexia, and tumor cell necrosis by angiogenesis inhibition, etc. That apoptosis of tumor cells does not make a contribution means that even when (-) -DHM2EQ is administered to the affected part, the effect does not depend on apoptosis of the tumor cells in the affected part. However, apoptosis may take place in tumor cells, apart from the effect on which apoptosis does not depend.

Each action and effect of (-)-DHM2EQ are explained in detail hereinbelow.

### Cell Adhesion-inhibitory Effect

When inflammatory, physical, and other stimuli are applied to vascular endothelial cells, expression of adhesion molecules on the vascular endothelial cell membrane is enhanced, and leukocytes adhere to the surface of the vascular endothelial cells to migrate out of blood vessels. This is because expression of adhesion molecules, such as ICAM-1, VCAM-1, and E-selectin, is enhanced by activation of NF-κB, a transcription factor, in vascular endothelial cells. Since adhesion of leukocytes to the blood vessel wall induces arteriosclerosis mediated by accumulation of lipids and other actions, it has long been considered that inhibition of adhesion between leukocytes and vascular endothelial cells leads to prevention/suppression of arteriosclerosis.

Further, it is known that adhesion of tumor cells causes their exudation and metastasis from blood vessels. For example, it has been reported that sialyl Lewis X, an E-selectin ligand, is highly expressed in high-metastasizing colon cancer, suggesting that activation of NF-κB in vascular endothelial cells enhances the expression of E-selectin on the vascular endothelial cell membrane. This facilitates adhesion of colon cancer cells to vascular endothelial cells and their exudation out of blood vessels and, accordingly, promoting metastasis.

If the cell adhesion activity of vascular endothelial cells can be inhibited, it may lead to prevention and suppression of arteriosclerosis or tumor metastasis. NF-κB inhibitors having such effects can be useful in preventing and suppressing arteriosclerosis or metastasis of tumor cells.

Thus, the inventors investigated the influence of a compound of formula (2) (hereinafter referred to as " (-)-DHM2EQ"), a compound of formula (4) (hereinafter referred to as " (+)-DHM2EQ"), and a compound of formula (3) (hereinafter referred to as " (+)-DHM2EQ"), all of which have been obtained by the above-mentioned manufacturing method, on the cell adhesion activity of human umbilical vein endothelial cells (HUVECs). The influence of each compound on the adhesion between vascular endothelial cells and tumor cells stimulated by TNF-α was examined. It was indicated that (-)-DHM2EQ inhibited the adhesion between vascular endothelial cells and tumor cells 10 times and 3 times as strongly as (+)-DHM2EQ and (±)-DHM2EQ did, respectively. These results revealed that (-)-DHM2EQ has a more potent inhibitory effect on adhesions of vascular endothelial cells than (±)-DHM2EQ. It is therefore concluded that (-)-DHM2EQ is more useful as a vascular endothelial cell adhesion inhibitor and also more useful as an anti-arteriosclerosis agent or a tumor metastasis suppressor than (±)-DHM2EQ.

### Anti-inflammatory Activity and Inhibitory Effect on Immunological Diseases

The inventors has clarified that administration of (±)-DHM2EQ to model mice of type II collagen-induced rheumatoid arthritis and observation of symptoms revealed efficacy of (±)-DHM2EQ in suppression of arthritic symptoms. This suggests that (±)-DHM2EQ is likely to exhibit an effect through the inhibition of the NF-κB activity. Accordingly, (-)-DHM2EQ, which has several to ten or more times stronger inhibitory effect on the NF-kappa B activity than (±)-DHM2EQ is thought to be even more effective as an anti-inflammatory agent in preventing/suppressing arthritic symptoms.

In conclusion, (-)-DHM2EQ can be expected to be a useful immunological disease suppressor and anti-inflammatory agent for symptoms of immunological diseases, inflammatory diseases (including allergic inflammatory diseases), etc.

### Inhibitory Effect on Tumor Cell Proliferation and Antitumor Effect

The inventors have recently clarified that (±)-DHM2EQ inhibits proliferation of Hodgkin's-lymphoma cells in which NF-κB is activated but that it does not inhibit proliferation of myelocytic leukemia cells in which NF-κB is not activated. This suggests that (±)-DHM2EQ is useful as a growth inhibitor of tumor cells in which NF-κB is activated and exerts an effect through suppression of NF-κB.

Further, the inventors have elucidated that (±)-DHN2EQ suppresses proliferation of human breast cancer cells in a manner not depending on apoptosis in vitro and in vivo. This suggests that (±)-DHM2EQ is useful as a preventive and therapeutic agent for breast cancer.

In addition, the inventors clarified that (-)-DHM2EQ suppresses tumor growth of in myeloma model mice (refer to Example 3). Multiple myeloma is accompanied by pains due to osteoclasis and causes excruciating pain; currently, no drastic treatment for multiple myeloma has been found. Accordingly, (-) -DHM2EQ is promising as an antitumor agent for myeloma such as multiple myeloma.

Moreover, the inventors have clarified that (-)-DHM2EQ suppresses tumor growth in thyroid cancer model mice (refer to Example 4) . Thyroid cancer is one of intractable cancers and currently no effective treatment for throid cancer has been found. Accordingly, (-)-DHM2EQ is promising as an antitumor agent for thyroid cancer.

Based on these findings, (-)-DHM2EQ, which has a stronger inhibitory effect on the NF-κB activation than (±)-DHM2EQ, can be expected to serve as an even more beneficial tumor cell proliferation inhibitor. It should be noted that tumor cells applied to the present invention include, but are not limited to, for example, thyroid cancer cells, prostatic cancer cells, breast cancer cells, malignant tumor cells, pancreatic cancer cells, etc. Any tumor cells can be applied as long as they are cells in which NF-κB are activated. As the aforementioned malignant tumor cells, preferred as an object of treatment are malignant tumor cells of the lymphoid system, among which Hodgkin's-lymphoma cells and myeloma cells are particularly preferred. Furthermore, since hormone-insensitive tumors that hormone therapy does not target can also be an object of treatment, this point is the great advantage over hormone therapy.

### Apoptosis-inducing, Anti-obesity, and Anti-insulin Resistance effects

The inventors have recently clarified that (±)-DHM2EQ induces apoptosis of leukemia cells in which NF-κB is activated. This indicates that (-) -DHM2EQ, which has a stronger inhibitory effect on NF-κB activation, can be expected to exert a superior apoptosis-inducing effect compared with (±)-DHM2EQ.

Conventional chemotherapy has extensively targeted proliferation mechanisms universal among cells; it has significantly affected not only tumor cells but also normal cells and hence it has not necessarily served as effective therapeutic means. In contrast, the inventors have obtained experimental results that (±)-DHM2EQ does not in the least induce apoptosis of human normal leukocytes at the same concentration as that used in inducing apoptosis of leukemia cells, revealing a high specificity of (±)-DHM2EQ to tumor cells. Accordingly, (-)-DHM2EQwith a higher specific activity probably has an even higher specificity to malignant tumor cells of the lymphoid system such as leukemia cells and has still fewer side effects on normal cells. Thus, the apoptosis inducer according to the present invention can be expected to exert a superior therapeutic effects as it can be suitably used for apoptosis induction in malignant lymphoma cells, leukemia cells, or myeloma cells and is highly specific to malignant lymphoma, leukemia, or myeloma.

Examples of the malignant tumors of the lymphoid system to be treated with the apoptosis inducer include malignant lymphoma, leukemia, or myeloma, etc. The aforementioned malignant lymphoma includes non-Hodgkin's lymphoma, Hodgkin's lymphoma, etc. The aforementioned myeloma includes plasma cell tumors etc., such as multiple myeloma. The aforementioned leukemia includes acute lymphatic leukemia, adult T-cell leukemia/lymphoma, chronic lymphocytic leukemia, etc.

By treating hypertrophic fat cells with (-)-DHM2EQ, apoptosis is induced. (-)-DHM2EQ can therefore be used as an apoptosis inducer for inducing apoptosis of hypertrophic fat cells.

Meanwhile, although the detailed mechanism has yet to be elucidated, it is known that fat cells that have become hypertrophic (large) due to obesity etc. secret TNF-α, a pro-inflammatory cytokine, into blood, which inhibits the insulin receptor-mediated signal transduction system, thereby resulting in insulin resistance (decreased insulin sensitivity). The insulin receptor-mediated signal transduction system also has the function of rapidly translocating the glucose transporter (GLUT), present within skeletal muscle cells in basal conditions, onto the plasma membrane, thereby promoting uptake of sugars (such as glucose) into skeletal muscle cells. It is thus considered that one cause of insulin resistance is the TNF-α inhibition of glucose uptake into cells by inhibiting the translocation of the glucose transporter onto the plasma membrane.

Therefore, if (-)-DHM2EQ induces apoptosis of hypertrophic fat cells, the level of TNF-α level secreted from fat cells into blood decreases, thereby preventing the inhibition of the action of intracellular glucose uptake. This and related effects not only make (-)-DHM2EQ effective as obesity preventive and inhibitory agent as well as a blood glucose level-lowering agent but also lead to improvement of symptoms of diseases involving insulin resistance or resulting from diabetes. Diseases involving insulin resistance illustratively include type 2 diabetes, hyperinsulinaemia, lipidosis, disorder, obesity, hypertension, arteriosclerotic diseases, etc. Diseases resulting from diabetes illustratively include diabetic nephropathy, diabetic retinopathy, diabetic neuropathy, etc. (-)-DHM2EQ can be expected to have beneficial therapeutic effects on these symptoms.

### Anti-ATL Effect

The inventors investigated the influence of (±)-DHM2EQ on NF-κB activation in adult T cell leukemia/lymphoma (ATL) cells and clarified that (±)-DHM2EQ inhibits NF-κB activation in ATL cells. Upon further investigation of the influence of (±)-DHM2EQ on proliferation of ATL cells, the inventors clarified that (±)-DHM2EQ does not inhibit proliferation of normal cells but are specifically capable of inhibiting proliferation of ATL cells. Yet another investigation of the influence of (±)-DHM2EQ on induction of apoptosis of ATL cells revealed that (±)-DHM2EQ does not induce apoptosis of normal cells but are capable of inducing apoptosis of ATL cells. These findings suggest that (±)-DHM2EQ has anti-ATL activity (effect).

It is thus concluded that (-)-DHM2EQ, which has a stronger inhibitory effect on NF-κB activation than (±)-DHM2EQ, is more useful as an anti-ATL agent.

### Anti-cachexia Effect

Cachexia is a disease that exhibits systemic defect with cardinal symptoms of anorexia, progressive loss of body weight, anemia, dry skin, edema, etc. in chronic diseases such as malignant tumors, tuberculosis, diabetes, hemopathy, and disorders of metabolism and internal secretion. Cachexia is often seen especially in terminally ill patients of malignant tumors etc. Patients with cachexia show loss of body weight, anemia, and other symptoms of deteriorations in systemic functions. Development of cachexia in cancer patients causes a high risk of complications and poor responses to chemotherapy. Moreover, weakness in the whole body produces strong side effects of chemotherapy or radiotherapy on cancer; cachexia can lead to death.

The detailed mechanism by which cachexia develops has not completely been elucidated yet. Only recently, however, have clues to the mechanism been emerging, including the involvement of cytokines such as interleukin-6 (IL-6) and tumor necrosis factor-α (TNF-α) (Saishin Igaku Vol.54, No. 10, 1999, 2502-2507) . For example, it is considered that the expression mechanism of various symptoms in cancer cachexia is the action of cytokines overexpressed due to induction of expression by cachexia on the central nervous system, resulting in symptoms such as decreased food intake, fever, low blood pressure, and the state of inertia, further leading to the enhancement state of sugar, protein, and lipid catabolism.

Administration of steroid is effective in suppressing such symptoms in cachexia. When steroid is administered to cachexia patients, the suppressive effect on immunological reaction, the resulting anti-inflammatory effect, and, further, the suppressive effect on the production of cachexia-inducing cytokines are exerted by steroid, whereby metabolic errors of cancer are corrected. As a result, cachexia symptoms such as loss of body weight, anorexia, inertia, dysgeusia, and anemia are alleviated and/or improved. However, long-term intake of steroid causes a problem of serious side effects. Since steroid is a hormone intrinsically present in the individual bodies, steroid taken in exhibits an active effect similar to that of an excess hormone, sometimes causing edema or high blood pressure as side effects by the involvement in the reabsorption of salt in the kidney.

Meanwhile, omega 3 unsaturated fatty acid suppresses the production of inflammatory cytokines such as IL-6 and influences the synthesis of acute phase reaction proteins. By taking advantage of these mechanisms, administration of eicosapentaenoic acid (EPA) has produced a certain effect in improving cachexia. However, since the action of such a nutrition formula is indirect, it is difficult to expect a marked and positive effect from that.

Therefore, there has been a growing demand for development of cachexia-specific drugs having a marked effect on cachexia, which are different from agents such as steroids having an extensive effect. In response to this request, the inventors administered (±)-DHM2EQ to model mice with induced cachexic symptoms and observed the symptoms, revealing efficacy of (±)-DHM2EQ in prevention/improvement of cachexic symptoms. It is thus concluded that (-)-DHM2EQ, which has a stronger inhibitory effect on NF-κB activation than (±)-DHM2EQ is more effective in preventing/improving cachexic symptoms.

### Suppressive Effect on Angiogenesis

Since (±)-DHM2EQ can inhibit activation of NF-κB, it follows that the composition can suppress gene expression of cyclooxygenase 2 (COX-2) that occurs by activation of NF-κB. Further, since (±)-DHM2EQ can suppress gene expression of cyclooxygenase 2, it follows that the compound can also suppress synthesis of prostaglandin downstream of COX-2. Within tumors, activation of NF-κB causes massive angiogenesis mediated by promotion of prostaglandin synthesis. It is suggested that (±)-DHM2EQ is capable of inhibiting tumor angiogenesis promoted by prostaglandin mediated by inhibition of NF-κB activation.

It is therefore thought that (±)-DHM2EQ is also useful as a cancer therapeutic agent that exerts an antitumor effect by inhibiting intratumoral angiogenesis and blocking supply of oxygen and nutrients to tumors.

It is thus concluded that (-)-DHM2EQ, which has a stronger inhibitory effect on the NF-κB activation than (±)-DHM2EQ is more effective as an intratumoral angiogenesis inhibitor.

### Anti-muscular Dystrophy Effect

The inventors clarified that the use of (-)-DHM2EQ makes it possible to relieve inhibition of differentiation induction from myoblasts to muscle cells. (-)-DHM2EQ can therefore be used as an agent for relieving inhibition of differentiation induction frommyoblasts to muscle cells. (-)-DHM2EQ is useful in improving muscular dystrophy that develops due to the inhibition of differentiation induction from myoblasts to muscle cells and can be expected to exert a superior therapeutic effect on it.

### Combined Effect of the Pharmaceutical Composition according to the Present Invention and the Therapy that Activates NF-κB

Traditionally, chemotherapy, radiotherapy, etc. are known as approaches for treating tumors. However, using chemotherapy and radiotherapy activates NF-κB in tumor cells, reducing the effect of cancer treatment (J.Clin. Invest.107, 241-246, 2001). For example, radiotherapy is performed in order to kill tumor cells, but irradiation-induced oxidative stress activates NF-κB, making it difficult for tumor cells to undergo apoptosis, resulting in resistance to radiotherapy. Thus, exposure of tumor cells to irradiation can diminish the effect of radiation. Accordingly, the development of a method for removing resistance to such cancer therapies is desired.

The inventors have recently clarified that use of (±)-DHM2EQ for oncotherapy that activates NF-κB, such as chemotherapy and radiotherapy, enhances the effect of this oncotherapy. Accordingly, it is suggested that (-)-DHM2EQ with a stronger inhibitory effect on NF-κB activation than (±)-DHM2EQ when used in combination with NF-κB-activating therapies, such as chemotherapy and radiotherapy, becomes useful in enhancing the effect these therapies.

The antitumor agent used for the above-mentioned chemotherapy is not limited as long as it is an antitumor agent that, at least, activates NF-κB. Such an antitumor agent illustratively include camptothecin (CPT) and daunomycin (generic name: daunorubicin; DNR or DM).

It should be noted that the agent to be used in combination with (-)-DHM2EQ is not limited to an antitumor agent as long as it activates NF-κB when administered and therefore produces resistance to a therapy. Illustratively, (-)-DHM2EQ can also be expected to increase therapeutic effects when used in combination with therapies for diseases such as infectious diseases, allergic diseases, immunological diseases, inflammatory diseases, tumor metastasis, cachexia, arteriosclerosis, neovascular diseases, (intratumoral neovascular disease in particular), leukemia (adult T-cell leukemia in particular), etc. In terms of treatment of administration, (-)-DHM2EQ may be administered simultaneously with an NF-κB-activating therapy, but it may be used to inhibit TNF-κB activation after the NF-κB-activating therapy. Alternatively, to inhibit activation of NF-κB, (-)-DHM2EQ may be administered in advance of the NF-κB-activating therapy. Patients to be reated include mammals including humans and animals other than humans afflicted with the above-mentioned diseases.

### === Method for Producing a Racemate of a Compound Represented by Formula (13)===

In the foregoing description, the method for producing optically active (-) and (+) compounds by directly resolving a racemate of a compound represented by formula (3) was explained. Similarly, it is possible to produce a (-) compound ((-)-DHM3EQ) represented by formula (14) 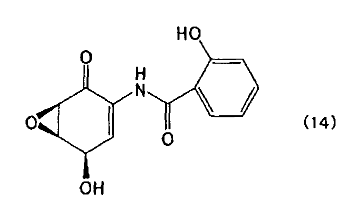 and a (+)- compound ((+)-DHM3EQ) represented by formula (15) 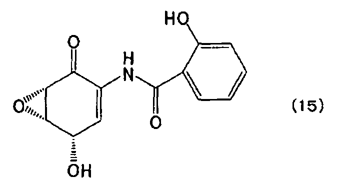 by directly resolving a racemate of a compound represented by formula (13).

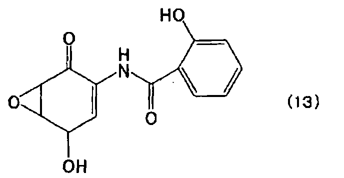

Method for producing a racemate of a compound represented by formula (13) is explained using the following reaction formula hereinbelow. In the following reaction formula, R² represents C1-4 alkyl group. Examples of the alkyl group include, a methyl group, an ethyl group, a propyl group, a butyl group, together with isomer groups thereof, and preferred among these are a methyl group and an ethyl group, particularly a methyl group.

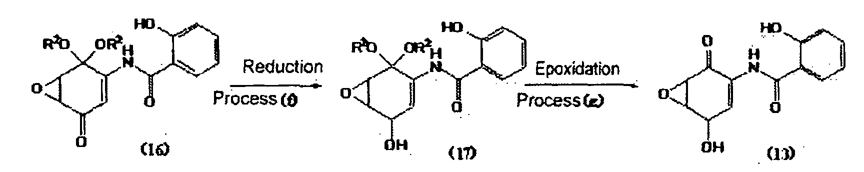

### Process f: Preparation of 3,3-dialkoxy-4,5-epoxy-6-hydroxy-2-salicyloylamino-cyclohexene

5,6-Epxoy-4,4-dialkoxy-3-salicyloylamino-2-cyclohexeno ne compound represented by formula (16) is dissolved in a mixed solution of a solvent such as methanol and sodium hydrogen carbonate solution, a reducing agent (sodium borohydride, etc.) is added at -78° C to 50°C, preferably under ice cooling, and the mixture is reacted while stirring. A solvent (ethyl acetate, etc.) is added to the reaction mixture, which is sequentially washed with hydrochloric acid and water. After drying, the solvent layer is concentrated under reduced pressure, dried under vacuum and purified by column chromatography to obtain 3,3-dialkoxy-4,5-epoxy-6-hydroxy-2-salicyloylamide-cyclohex ene represented by formula (17)

### Process g: Preparation of 5,6-epoxy-4-hydroxy-2-salicyloylamino-2-cyclohexenone (Formula (13), DHM3EQ)

3,3-Dialkoxy-4,5-epoxy-6-hydroxy-2-salicyloylamino-cy clohexene represented by formula (17) is dissolved in a solvent (acetone, etc), p-toluenesulfonic acid is added to the solution, which is then reacted at room temperature while stirring. A solvent (ethyl acetate, etc.) is added to the reaction mixture, which is washed with water. The solvent layer is dried, concentrated under reduced pressure and purified to obtain 5,6-epoxy-4-hydroxy-2-salicyloylamino-2-cyclohexenone (DHM3EQ) represented by formula (13).

(±)-DHM3EQ thus obtained can be resolved into (+)-DHM3EQ and (-)-DHM3EQ directly by using an optically active column packed with a resolving agent containing as an active ingredient a polysaccharide aromatic carbamate derivative substituted with a group represented by formula (5) or by using the aforementioned resolving agent in a batch system. The preferred optically active column to be used is one with which (±)-DHM2EQ can be directly resolved into (+)-DHM2EQ and (-)-DHM2EQ. The preferred resolving agent to be used is one with which (±)-DHM2EQ can be directly resolved into (+)-DHM2EQ and (-)-DHM2EQ.

It should be noted that (-)-DHM3EQ (formula (14)) or (+)-DHM3EQ (formula (15)) obtained by purification in the aforementioned manner has a more beneficial pharmacological effect than their racemic compound, like (-)-DHM2EQ, and is thought to be useful in improving symptoms accompanied by activation of NF-κB.

### === Other Embodiments ===

In the foregoing description, the pharmacological effect of the compound in which R¹ of a compound represented by formula (1) is an acetyl group. However, it is suggested that the compound in which R¹ of a compound represented by formula (1) is a hydrogen atom, a propionyl group, a butanoyl group, or isomer groups thereof has a similar pharmacological effect.

It should be noted that the compound according to the present invention may be in the form of a pharmacologically acceptable salt such as an organic salt (e.g., quaternary ammonium salt etc.) or a metal salt (e.g., alkali metal) . Salts of such compounds can be produced by known methods.

### Example 1

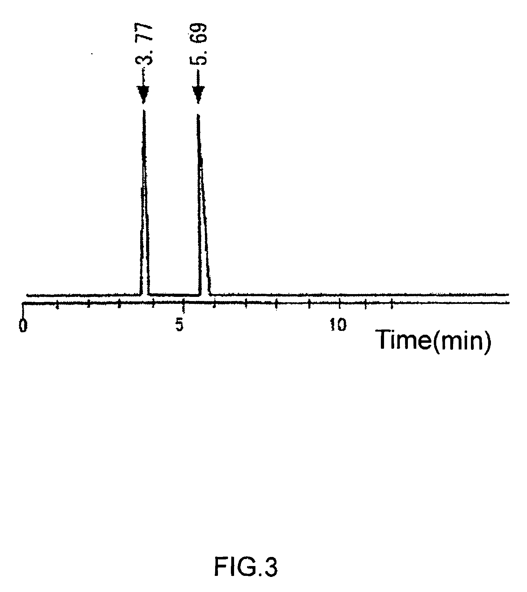

(±)-5-Dehydroxymethyl epoxyquinomicin C (dehydroxymethyl derivatives of epoxyquinomicin C; DHM2EQ) was synthesized using the methods described above. (±)-DHM2EQ obtained was optically resolved by high performance liquid chromatography (HPLC; detection wavelength, 315 nm UV; column temperature, 40°C) on a DADAICEL CHIRALPAK AD (10 mm i.d. x 250 mm) using 0.5 v/v% acetic acid in methanol as a mobile phase (at a flow rate of 1.0 ml/min) . Twenty milligram of (±)-DHM2EQ was dissolved in 2 ml of methanol. The solution was injected in 20 portions (100 µl aliquots) and isolated at 3.8 min and 5.7 min peaks to give (-)-DHM2EQ and (+)-DHM2EQ at a recovery rate of 85%. FIG. 3 shows the separation profile.

The optical rotations of the two resulting compounds ((-)-DHM2EQ: [α]²⁰_{D} -241° (c0.1 MeOH) , (+)-DHM2EQ: [α]²⁰_{D} +239° (c0.1 MeOH)) corresponded with the optical rotations described in literature (Bioorg. Med. Chem. Lett. 10, 865-869, 2000) with an optical purity (ee) of 99% or higher.

### Example 2

In this example, the effects of (-)- DHM2EQ and (+)-DHM2EQ on adhesion between vascular endothelial cells and tumor cells stimulated by TNF-α were investigated.
(1) Cell Culture
   Normal human umbilical vein endothelial cells (HUVECs; Cell systems, Lake Kirland, WA) were incubated at 37°C in a 5% CO₂ incubator, using plastics flasks (Costar, New York, USA) coated with type I collagen (Koken Co., Ltd., Tokyo, Japan), in MCDB-131 medium (Sigma, St.Louis, MO) containing 10 µg/ml bFGF (Pepro Tech EC LTD, London, UK) supplemented with heat-inactivated 10% fatal bovine serum (FBS; JRH BIOSCIENCES Lenexa, KS). Human acute promyelocytic leukemia cell line HL-60 cells (Japanese Collection of Research Bioresources) were incubated at 37 °C in a 5% CO₂ incubator using RPMI1640 medium (NISSUI PHARMACEUTICAL CO., LTD., Tokyo, Japan) supplemented with heat-inactivated 10% FBS.
(2) Cell Adhesion Experiment

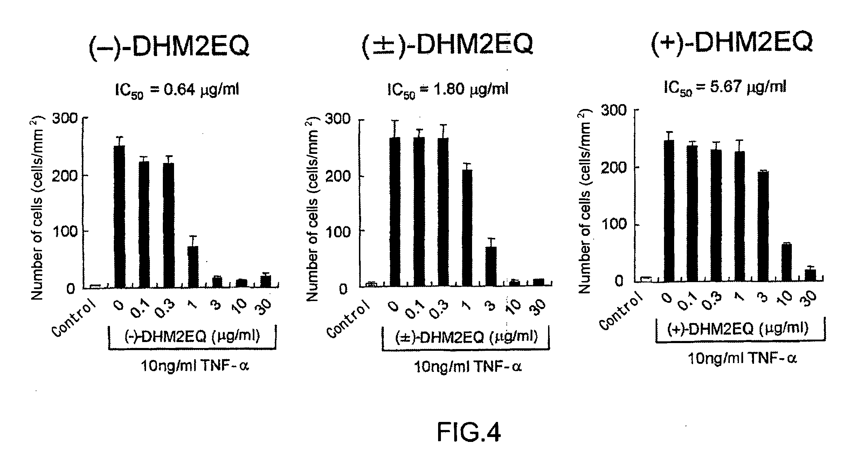

HUVECs were plated at 4.0 x 10⁴ cells/well (500 µl/well) in 48-well plates (Costar) and incubated overnight. On the next day, cultured HUVECs were treated with (-)-DHM2EQ, (±)-DHM2EQ, or (+)-DHM2EQ, diluted to various concentrations, and incubated for 2 h at 37°C in 5% CO2. Subsequently, 10 ng/ml TNF-α Genzyme-Techne, Cambridge, MA) was added, followed by incubation for 2 h at 37 °C in 5% CO₂. (TNF-α-untreated cells were used as controls.) After 6 h, each well was washed twice with pre-warmed (37°C) phosphate buffered saline (PBS) and then replenished with a fresh medium (200 µl/well) . Subsequently, each well was plated with HL-60 cells at a concentration of 6.0 x 10⁴ cells/well (20 µl/well), followed by incubation for 1 h at 37°C in 5% CO₂. Next, non-adhered HL-60 cells were removed by washing each well three times with pre-warmed (37°C) PBS and a fresh medium (200 µl/well) was added. Subsequently, HL-60 cells adhered to HUVECs were confirmed with a microscope, and their adhesion ability was evaluated by counting the number of adherent cells in the microscopic field. The results, together with the IC₅₀ value of each DHM2EQ, are shown in FIG. 4. FIG. 4 indicates that the cell adhesion inhibitory activity of (-)-DHM2EQ is ten times and three times as strong as those of (+)-DHM2EQ and (±)-DHM2EQ, respectively.

### Example 3

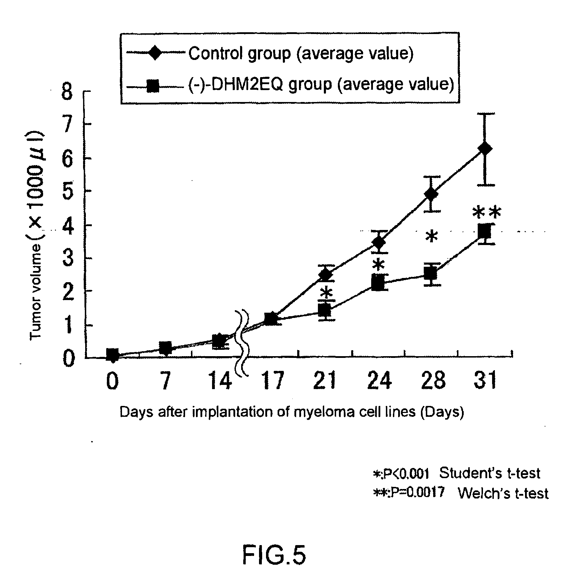

Next, the antitumor effect of (-)-DHM2EQ on multiple myeloma was investigated. The multiple myeloma cells line KMS-12-PE (Ohtsuki T. et al., Br. J. Hematol. 73, 199-204, 1989) (4 x 10⁷ cells) was dissolved in 0.2 ml of PBS and injected subcutaneously into the left back of CB17/lcr-SCID mice to form tumors. Pieces of these tumors (5 mm³) were implanted in 10 mice. After the implantation of tumors, the tumor grafts were observed for 16 days and their survival and proliferation tendency were confirmed. Subsequently, a solution of (-)-DHM2EQ (RPMI1640 medium) was administered intraperitoneally to 5 mice at 8 mg/kg BW every day for 14 days ((-)-DHM2EQ) . Another five mice in the control group were given an equal amount of solvent (Control) . The diameters of tumors were measured twice a week (Tumor volume = (minor axis)² x (major axis) x 0.52) . The results are shown in FIG. 5. It was revealed that (-)-DHM2EQ significantly suppresses tumor growth compared with the control.

### Example 4

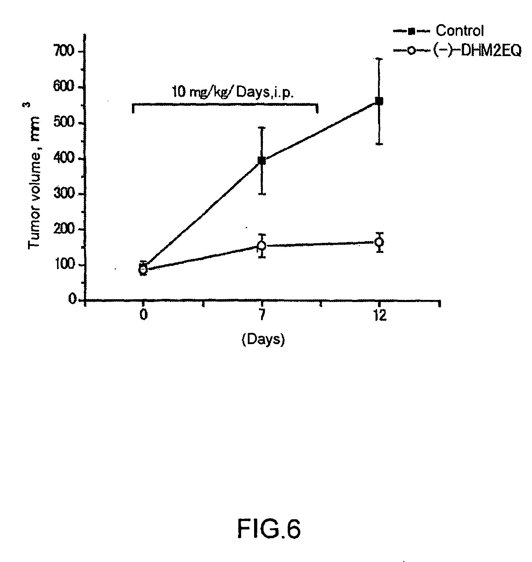

The antitumor effect of (-)-DHM2EQ on human thyroid cancer was investigated. Undifferentiated human thyroid cancer cell suspension FRO (5 x 10⁶ cells; 1200 µl of RPMI-1640) was injected subcutaneously into the flank of 8-week-old BALB/cnu/nu male mice (Charles River, Japan). Subsequently, tumor sizes were measured with calipers every other day, and tumor volume was calculated by formula (a; minor axis of tumor)² x (b: perpendicular diameter to a) x 0.4). Starting from the day (day 0) when the tumor volume reached 100 mm³, which was 14 days after subcutaneous injection, a solution of (-)-DHM2EQ ((Cremophor (BASF Co.):PBS:DMSO (2:1:1)) was administered intraperitoneally to a group of 5 mice at 10 mg/kg BW every day for 10 days (o: (-)-DHM2EQ) . Another nine mice in the control group were given an equal amount of solvent (■: Control). The results are shown FIG. 6. It was revealed that (-)-DHM2EQ significantly inhibits tumor growth compared with the control.

### Example 5

It is considered that diseases involving insulin resistance result from TNF-α secreted from hypertrophic fat cells, as mentioned above. Thus, to examine whether (-)-DHM2EQ can induce apoptosis of hypertrophic fat cells, the influence of (-)-DHM2EQ on the hypertrophic fat cells obtained by inducing differentiation of precursor fat cells in long-term culture was investigated. The precursor fat cell line 3T3-L1 (7.5 x 10⁴ cells; 1 ml of DMEM + 10% FBS) was plated on coverslips in 12-well plates. A few days after the cells reached confluency, the cell medium was changed to a medium containing 500 µM of 3-isobutyl-1-methylxanthine (IBMX), 1 µM of dexamethasone (Dex), and a medium containing 1 µg/ml of insulin. After culture for 3 days, this medium was changed to a media containing 1 µg/ml insulin, which was cultured for another 3 days, followed by replacement with a normal medium (DMEM containing 10% FBS) . Thereafter, medium change was performed every three days continuously till day 50.

On day 50 after differentiation induction, medium change was performed. After 24 h, hypertrophic fat cells were pretreated with (-)-DHM2EQ (10 µg/ml)) for 30 min and subsequently stimulated with TNF-α (10 ng/ml) further added. (The experiment was carried out under the conditions in which cells without (-)-DHM2EQ, cells without TNF-α, and cells without (-)-DHM2EQ or TNF-α were used as controls.). After another 24 h, the medium was removed and hypertrophy fat cells were washed 3 times with 500 µl of PBS (8 g/l NaCl, 0.45 g/l NaH₂PO₄.2H₂O, 1.28 g/l Na₂HPO₄) for fluorescent antibodies and fixed for 15 min at 4°C in 500 µl of 4% paraformaldehyde. Subsequently, cells were washed three times with 500 µl of PBS for fluorescent antibodies and treated for 15 min with 0.2% BSA and 0.5% Tween-20 in PBS for fluorescent antibodies.

Subsequently, the coverslips were washed three times with 500 µl of distilled water and then mounted on the paraffin sheet in 150-mm² shielded Petri dishes, cells facing upward. TUNEL solution (TdT buffer II/FITC-dUTP/TdT = 45 µl/2.5 µl/2.5 µl) was dropped in 45-µl drops onto the coverslips, followed by incubation for 1 h at 37°C. Finally, the coverslips were returned to shielded 12-well plates and washed three times with 1 ml of PBS for fluorescent antibodies. Then the coverslips were removed, mounted on a slide in 50% glycerol, and observed under a fluorescence microscope.

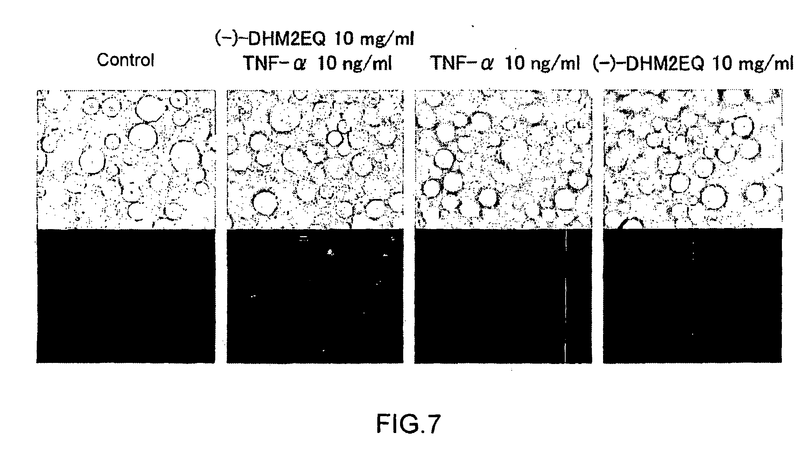

The results are shown in FIG. 7. It was revealed that the hypertrophic fat cells pretreated with 10 mg/ml (-) -DHM2EQ induces apoptosis by stimulation with 10 ng/ml TNF-α. The hypertrophic fat cells treated either with 10 ng/ml TNF-α or with 10 mg/ml(-)-DHM2EQ did not induce apoptosis.

In conclusion, it is suggested that (-) -DHM2EQ is useful in the improvement (including prevention and treatment) of the insulin resistance syndromes resulting from TNF-α-secreting hypertrophic fat cells (e.g., type 2 diabetes, hyperinsulinaemia, lipidosis, obesity, hypertension, arteriosclerotic diseases, etc.) . It is further suggested that (-)-DHM2EQ is also useful as obesity-preventive agent, a diet drug, and a blood glucose level-lowering agent. Moreover, it is suggested that (-)-DHM2EQ is also useful in the improvement (including prevention and treatment) of diseases resulting from diabetes (e.g., diabetic nephropathy, diabetic retinopathy, diabetic neuropathy, etc.).

### Example 6

Next, the effect of (-)-DHM2EQ on diabetes was investigated using KKA^{y} mice, a model for obese type 2 diabetes.

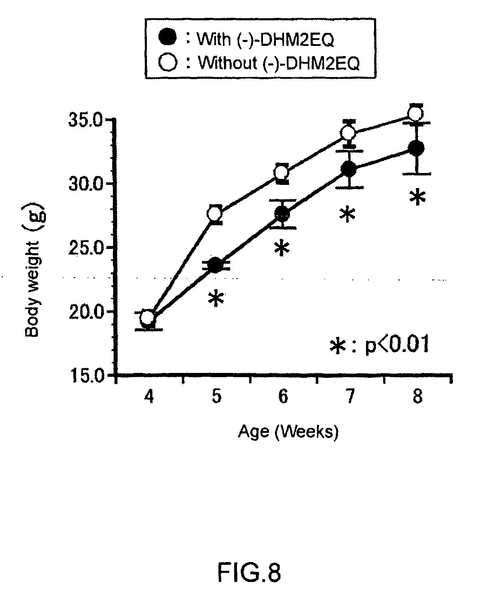

Four-week-old KKA^{y} female mice were divided into two groups: the (-)-DHM2EQ group (n = 5) and the control group (n = 4). A high fat diet (CRF-1; Oriental Yeast Co.) as a basal chow diet was fed ad libitum for 4 weeks to the non-supplemented group (o: without (-)-DHM2EQ) and the (-)-DHM2EQ-administered group (●: with (-)-DHM2EQ). Body weights of animals were measured weekly. The (-)-DHM2EQ given to mice was ground to fine powder in a mortar, suspended or dissolved in a 5% CMC solution, and supplemented to the high fat diet. The results are shown in FIG. 8. The non-supplemented group showed a marked weight gain resulting from a high fat diet load, whereas the (-)-DHM2EQ group showed suppression of body weight even under the high fat diet. In conclusion, it is suggested that (-)-DHM2EQ is useful as an anti-obesity agent (an obesity preventive and inhibitory agent, including a diet drug) and an antidiabetic agent.

### Example 7

Progressions of muscular dystrophies in muscular dystrophy patients, including the Duchenne-type, are considered to be caused partly by the mechanism of muscle cell degeneration resulting from inhibition of differentiation induction from myoblasts to myocyts (Igaku no Ayumi, 199: 1045-1048, 2001). In mdx mice, a model of Duchenne muscular dystrophy, in spite of the lack of dystrophin expression, like in human Duchenne muscular dystrophy patients, active muscle regeneration virtually prevents the development of muscle weakness. This is probably because, in human patients, inflammatory factors such as TNF-α inhibit muscle differentiation in the process of regeneration of muscle cells once destroyed.

Thus, the inventors investigated whether addition of (-)-DHM2EQ can relieve the inhibitory responses to muscle differentiation by TNF-α, using a differentiation induction system of the C2C12 myoblast cell line. Using DMEM (culture medium) containing 10% FBS a mouse skeletal myogenic cell line, C2C12 (purchased from the RIKEN cell bank) was prepared at 7.5 x 10⁴ cells/ml. Cells were seeded in plastic 12-well plates on coverslips (1 ml/well; Matsunami Glass Ind., Ltd., Osaka, Japan). Subsequently, when cells had grown to confluence, the medium was changed to DMEM (differentiation medium) containing 0.5% heat-inactivated horse serum. Cells to be subjected to the effect of TNF-α (1 ng/ml) and/or (-)-DHMEQ (3 µg/(ml)) were supplemented with each agent at this point. After 6 days, on confirmation of the formation of multinucleated myotubes, a marker of muscle cell differentiation, the medium was removed. The control was prepared by culturing cells in a culture medium at a density such that confluency was reached exactly on day 6.

These cells were washed twice with PBS⁻ (Ca²⁺, Mg²⁺-free PBS; 8.0 g/l NaCl, 0.2 g/l KCl, 2.3 g/l NaHPO₄.12H₂O, 0.2 g/l KH₂PO₄) . Subsequently, the coverslips were removed from the wells and dried with a cold air dryer. Then, the coverslips were placed in the 12-well plates again, to which May-Grünwald solution was added (300 µl/well) . The cells submerged in the solution on the coverslips were allowed to stand for a few minutes before fixation and staining. After a few minutes, cells were further fixed and stained for another few minutes by quickly adding an equal volume of PBS⁻, pH 6.7, to each well, with air bubbled into the solution in each well. The coverslips were removed from the wells, then washed with water, and dried by cool air from a dryer.

The coverslips were placed in 12-well plates, to which Giemsa staining solution (a solution composed of 1 m of PBS⁻ (pH: 6.7) and 45 µl of Giemsa staining solution (Merck)) was added to submerge the coverslips. Subsequently, the nuclei were stained for 10 to 15 min, with air bubbled into the solution in each well again. The coverslips were then removed, washed with water, and dried from cool air from a dryer in the same manner as described above.

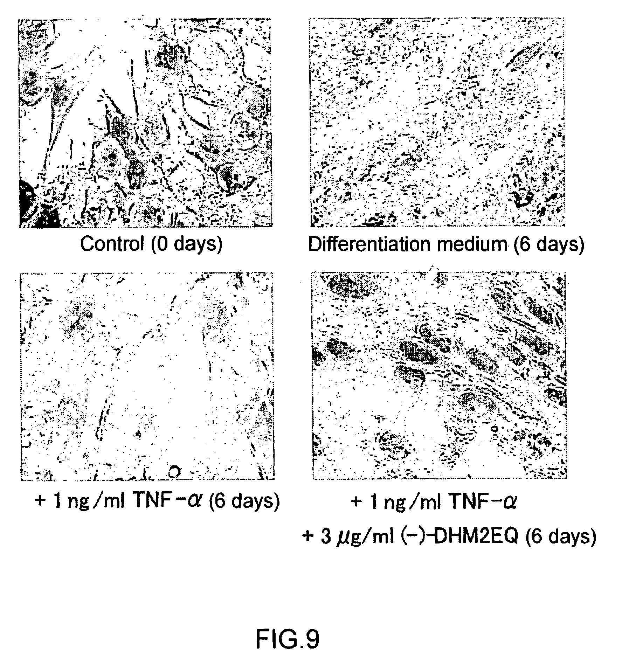

Samples thus prepared were mounted on a slide and photographed at 400x magnification with a fluorescence microscope (Nikon UFX-DX; set with a 40X phase contrast objective, Ph2). The results are shown in FIG. 9. It was revealed that 1 ng/ml TNF-α suppressed differentiation induction from myoblasts to muscle cells, whereas 1 to 3 µg/ml (-)-DHM2EQ relieves the inhibition of the differentiation induction. It should be noted that the differentiation rate of 2CC12 cells which had not been induced to differentiate was 0%.

In conclusion, it is expected that by administering (-)-DHM2EQ to muscular dystrophy patients, including the Duchenne-type, inhibition of muscle differentiation by TNF-α is relieved, whereby muscle regeneration is induced, leading to the improvement of muscular dystrophy diseases.

### Industrial Applicability

According to the present invention, pharmaceutical compositions useful for symptoms, such as immunological diseases, allergic diseases, inflammatory diseases, tumor metastasis, cachexia, arteriosclerosis, etc; methods for producing optically active compounds to be contained as active ingredients in the pharmaceutical composition; and direct resolution methods for obtaining such compounds can be provided.

## Claims

1. A pharmaceutical composition for improving a symptom accompanied by activation of NF-κB, comprising an optically active compound represented by the following general formula (1) or a pharmacologically acceptable salt thereof as an active ingredient. 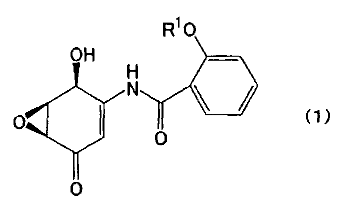 wherein R¹ represents a hydrogen atom or a C2-4 alkanoyl group.

2. The pharmaceutical composition of claim 1, wherein the compound is the following formula (2).

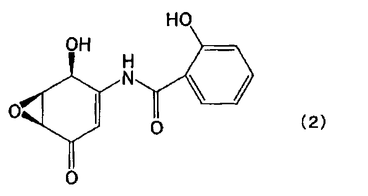

3. The pharmaceutical composition of claim 1 or 2, wherein the symptom results from a tumor cell.

4. The pharmaceutical composition of claim 3, which improves the symptom by inhibiting proliferation of the tumor cell.

5. The pharmaceutical composition of claim 3, which improves the symptom by inhibiting cell adhesion activity of a vascular endothelial cell.

6. The pharmaceutical composition of claim 1, wherein the symptom is one selected from the group consisting of an immunological disease, an allergic disease, an inflammatory disease, tumor metastasis, cachexia, arteriosclerosis, and leukemia.

7. A pharmaceutical composition comprising as an active ingredient an optically active compound represented by the following general formula (1) or a pharmacologically acceptable salt thereof, which is capable of enhancing the effect of a therapy by inhibiting activation of NF-κB caused by the therapy that causes the activation of NF-κB. 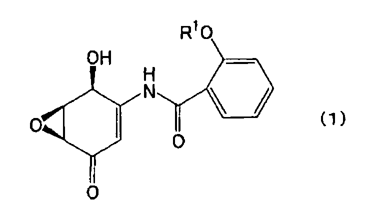 wherein R¹ represents a hydrogen atom or a C2-4 alkanoyl group.

8. The pharmaceutical composition of claim 7, wherein the compound is the following formula (2) .

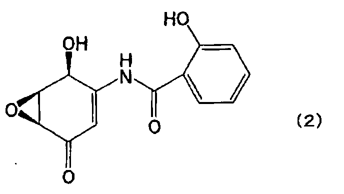

9. The pharmaceutical composition of claim 7 or 8, wherein the therapy that activates NF-κB is a therapy using an antitumor agent.

10. The pharmaceutical composition of claim 7 or 8, wherein the therapy that activates NF-κB is radiotherapy for a tumor cell.

11. The pharmaceutical composition of claim 9, comprising the antitumor agent as an active ingredient.

12. The pharmaceutical composition of claim 9, wherein the antitumor agent is camptothecin or daunorubicin.

13. A pharmaceutical composition for improving a disease caused by TNF-α, comprising an optically active compound represented by the following general formula (1) or a pharmacologically acceptable salt thereof as an active ingredient. 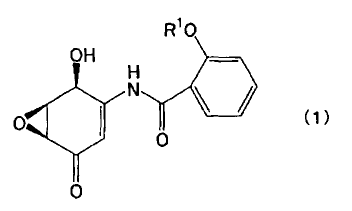 wherein R¹ represents a hydrogen atom or a C2-4 alkanoyl group.

14. The pharmaceutical composition of claim 13, wherein the compound is the following formula (2).

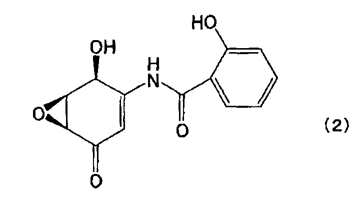

15. The pharmaceutical composition of claim 13 or 14, wherein the disease caused by TNF-α is a disease involving insulin resistance.

16. The pharmaceutical composition of claim 13 or 14, wherein the disease caused by TNF-α is a disease resulting from diabetes.

17. The pharmaceutical composition of claim 13 or 14, wherein the disease caused by TNF-α is a muscular dystrophy.

18. A tumor cell proliferation inhibitor for inhibiting proliferation of a tumor cell, comprising an optically active compound represented by the following general formula (1) or a pharmacologically acceptable salt thereof as an active ingredient. 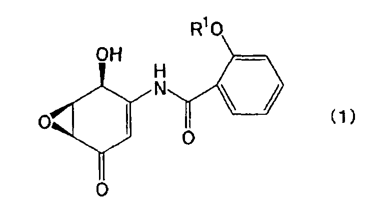 wherein R¹ represents a hydrogen atom or a C2-4 alkanoyl group.

19. The tumor cell proliferation inhibitor of claim 18, wherein the compound is the following formula (2).

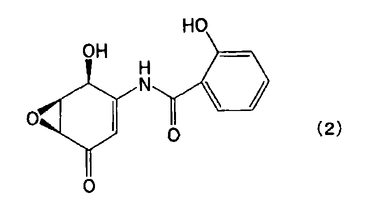

20. A cell adhesion inhibitor for inhibiting cell adhesion of a vascular endothelial cell, comprising an optically active compound represented by the following general formula (1) or a pharmacologically acceptable salt thereof as an active ingredient. 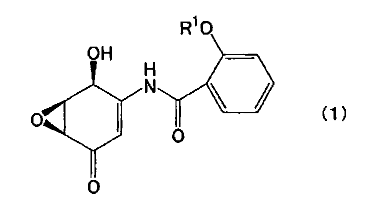 wherein R¹ represents a hydrogen atom or a C2-4 alkanoyl group.

21. The cell adhesion inhibitor of claim 20, wherein the compound is the following formula (2).

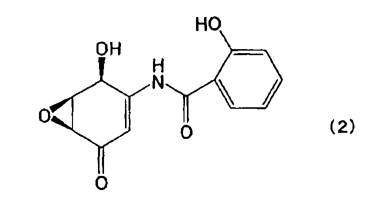

22. An apoptosis inducer for inducing apoptosis of a tumor cell, comprising an optically active compound represented by the following general formula (1) or a pharmacologically acceptable salt thereof as an active ingredient. 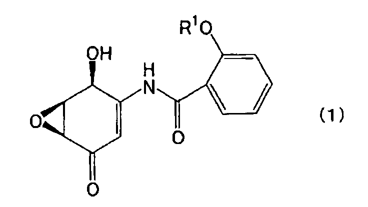 wherein R¹ represents a hydrogen atom or a C2-4 alkanoyl group.

23. The apoptosis inducer of claim 22, wherein the compound is the following formula (2).

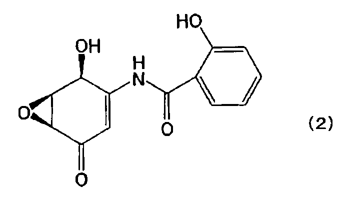

24. An apoptosis inducer for inducing apoptosis of a hypertrophic fat cell, comprising an optically active compound represented by the following general formula (1) or a pharmacologically acceptable salt thereof as an active ingredient. 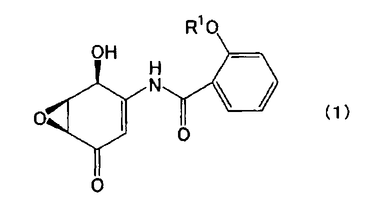 wherein R¹ represents a hydrogen atom or a C2-4 alkanoyl group.

25. The apoptosis inducer of claim 24, wherein the compound is the following formula (2).

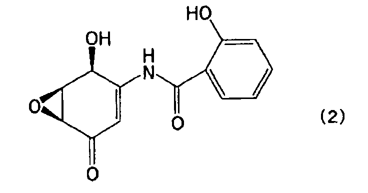

26. The apoptosis inducer of claim 24 or 25, further comprising TNF-α as an active ingredient.

27. An obesity preventive and inhibitory agent comprising an optically active compound represented by the following general formula (1) or a pharmacologically acceptable salt thereof as an active ingredient. 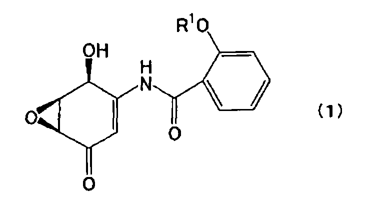 wherein R¹ represents a hydrogen atom or a C2-4 alkanoyl group.

28. The obesity preventive and inhibitory agent of claim27, wherein the compound is the following formula (2).

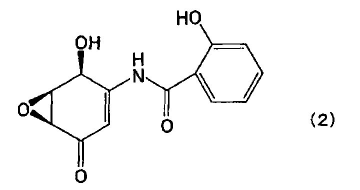

29. A blood glucose level-lowering agent comprising an optically active compound represented by the following general formula (1) or a pharmacologically acceptable salt thereof r as an active ingredient. 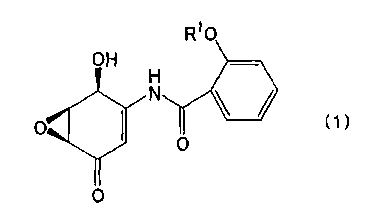 wherein R¹ represents a hydrogen atom or a C2-4 alkanoyl group.

30. The obesity preventive and inhibitory agent of claim 29, wherein the compound is the following formula (2).

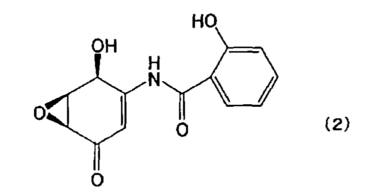

31. An agent for relieving inhibition of induction of cell differentiation comprising an optically active compound represented by the following general formula (1) or a pharmacologically acceptable salt thereof as an active ingredient. 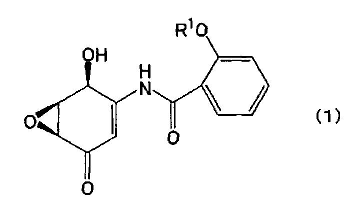 wherein R¹ represents a hydrogen atom or a C2-4 alkanoyl group.

32. The agent for relieving inhibition of induction of cell differentiation of claim 31, wherein the compound is the following formula (2) .

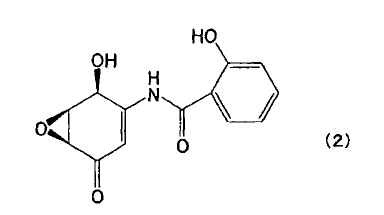

33. An agent for relieving inhibition of induction of cell differentiation of claim 31 and 32, wherein the inhibition of induction of cell differentiation is suppression of muscle cell differentiation by TNF-α.

34. A method for producing an optically active compound represented by the following formula (2) 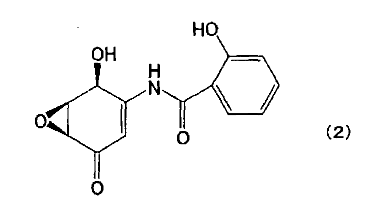 or (4) 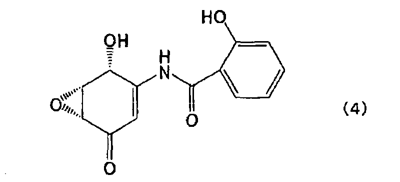 wherein a racemate of a compound represented by formula (3) 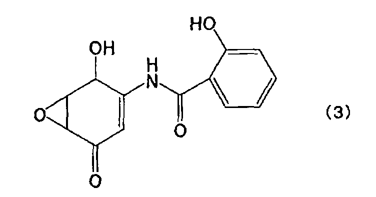 is directly optically resolved.

35. A method of claim 34, wherein the optical resolution is performed using an optically active column.

36. The method of claim 35, wherein the optically active column is packed with a resolving agent containing as an active ingredient a polysaccharide aromatic carbamate derivative substituted with a group represented by the following formula (5). 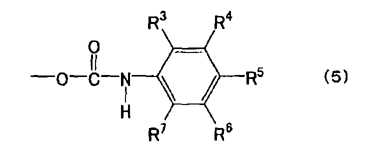 wherein R³ to R⁷ each individually represent hydrogen atoms or alkyl groups having 1 to 8 carbon numbers.

37. The methods of claim 36, wherein the polysaccharide aromatic carbamate derivative is amylose tris (3,5-dimethyl phenylcarbamate).

38. A direct resolution method comprising directly optically resolving a racemate of a compound represented by formula (3) 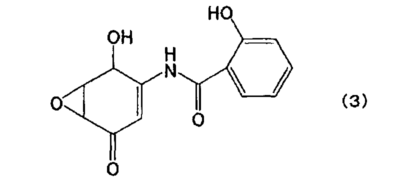 using a resolving agent containing as an active ingredient a polysaccharide aromatic carbamate derivative substituted with a group represented by the following formula (5). 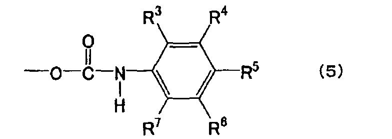 wherein R³ to R⁷ each individually represent hydrogen atoms or alkyl groups having 1 to 8 carbon numbers.

39. The direct resolution method of claim 38, wherein the polysaccharide aromatic carbamate derivative is amylose tris (3, 5-dimethylphenylcarbamate) .

40. A therapeutic method comprising using an optically active compound represented by the following general formula (1) or a pharmacologically acceptable salt thereof for improving a disease accompanied by activation of NF-κB. 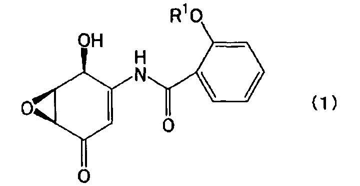 wherein R¹ represents a hydrogen atom or a C2-4 alkanoyl group.

41. The therapeutic method of claim 40, wherein the compound is the following formula (2).

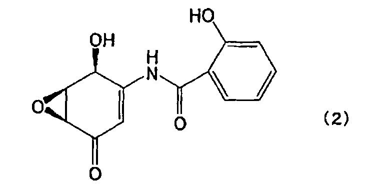

42. The therapeutic method of claim 40 or 41, wherein the symptom results from a tumor cell.

43. The therapeutic method of claim 42, which improves the symptom by inhibiting proliferation of the tumor cell.

44. A therapeutic method of claim 40, which improves the symptom by inhibiting cell adhesion activity of a vascular endothelial cell.

45. The therapeutic method of claim 40, wherein the symptom is one selected from the group consisting of an immunological disease, an allergic disease, an inflammatory disease, tumor metastasis, cachexia, arteriosclerosis, and leukemia.

46. A therapeutic method comprising the steps of performing a therapy for activating NF-κB and administering a pharmaceutical composition containing as an active ingredient an optically active compound or a pharmacologically acceptable salt thereof represented by the following general formula (1) . 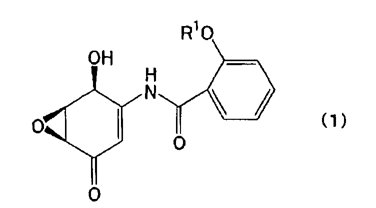 wherein R¹ represents a hydrogen atom or a C2-4 alkanoyl group.

47. The therapeutic method of claim 46, wherein the compound is the following formula (2).

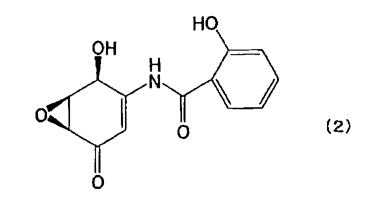

48. The therapeutic method of claim 46 or 47, wherein the therapy that activates NF-κB is a therapy using an antitumor agent.

49. The therapeutic method of claim 46 or 47, wherein the therapy that activates NF-κB is radiotherapy for a tumor cell.

50. A therapeutic method comprising using an optically active compound represented by the following general formula (1) or a pharmacologically acceptable salt thereof for improving a disease caused by TNF-α. 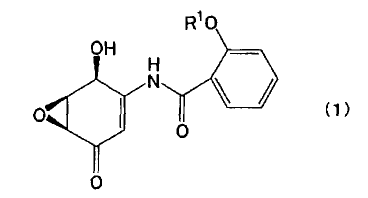 wherein R¹ represents a hydrogen atom or a C2-4 alkanoyl group.

51. The therapeutic method of claim 50, wherein the compound is the following formula (2).

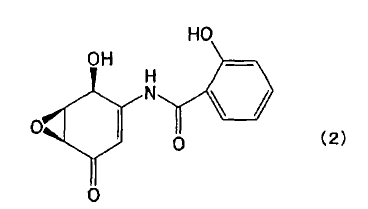

52. The therapeutic method of claim 50 or 51, wherein the disease caused by TNF-α is a disease involving insulin resistance.

53. The therapeutic method of claim 50 or 51, wherein the disease caused by TNF-α is a disease resulting from diabetes.

54. The therapeutic method of claim 50 or 51, wherein the disease caused by TNF-α is a muscular dystrophy.
